# EUROPEAN PATENT APPLICATION

(11) **EP 1 745 736 A1**
(43) Date of publication of application: **24.01.2007**
(21) Application number: 05736757.5
(22) Date of filing: 28.04.2005
(51) Int. Cl.: A61B 1/00, A61B 1/24

(54) **LIVING BODY OBSERVING APPARATUS, INTRAORAL IMAGING SYSTEM, AND MEDICAL TREATMENT APPLIANCE**

(30) Priority: 30.04.2004 JP 2004136008
(71) Applicant: J. Morita Manufacturing Corporation, Fushimi-ku, Kyoto-shi Kyoto 6128213 (JP)
(72) Inventor: OKAWA, Shinichi, J. MORITA MANUFACTURING CORP., Kyoto-shi, Kyoto 6128213 (JP); KINO, Kenji, J. MORITA MANUFACTURING CORP., Kyoto-shi, Kyoto 6128213 (JP); MATOBA, Kazunari, J. MORITA MANUFACTURING CORP., Kyoto-shi, Kyoto 6128213 (JP)
(74) Representative: Hoffmann, Jörg Peter
(86) International application number: PCT/JP2005/008218
(87) International publication number: WO 2005/104926

(57) **Abstract**

A medical living body observation apparatus, an intraoral photography system and a medical treatment appliance which can be used conveniently by a general user at home as well as a technician, e.g. a dentist or a physician. There is provided an irradiation means (2) comprising an exciting light emission section (2a) emitting an exciting light for causing a lesion to radiate fluorescence, and an illumination light emission section (2b) emitting a light for illuminating the periphery of the lesion. The irradiating means (2) can simultaneously irradiate the illumination light and the exciting light.

## Description

### TECHNICAL FIELD

The present invention relates to a living body observing apparatus including a medical observing apparatus for an operator to use in diagnosis of: caries condition of a tooth, a missing part, or lesion, deposition condition of tartar or plaque, or lesion of root canal, gum, cheek, or tongue in the oral cavity; or in diagnosis of otological regions; treatment and diagnosis of rectal abscess and the like; or dermatological diagnosis. The present invention further relates to an intraoral imaging apparatus capable of sharply visually recognizing and further imaging the condition of the lesion. Moreover, the present invention relates to a living body observing apparatus, an intraoral imaging apparatus, and a medical treatment appliance which permit a general user to, for example, observe his (her) or his (her) family member's tartar deposition condition, progress of caries of a tooth in the oral cavity, and further skin condition.

### BACKGROUND ARTS

For example, an intraoral imaging apparatus for intraoral diagnosis, which needs to be inserted and operated in the mouth, is required to have a portion of imaging means compactly structured. That is, conventionally, such the intraoral imaging apparatus has, at a tip end part of the main body gripped and supported by hands and fingers, a light source for irradiating light to an object to be diagnosed, CCD imaging means, and the like, which are so constructed as to be arranged together in an extremely compact manner. For example, Patent document 1 indicates well-known structure of this apparatus.

The intraoral imaging apparatus according to conventional art described in the patent document 1 is constructed to be elongated as a whole, with the tip end part thereof shaped thin, and thus can be easily inserted in the oral cavity, and it is also capable of performing imaging while brightly illuminating the inside of the oral cavity by providing a white LED as an illumination light, thus carrying the advantage of being capable of easily imaging a desired area in the narrow oral cavity. However, the capability of this intraoral imaging apparatus is limited to imaging a tooth or the surface condition in the oral cavity under the irradiation of a visible light (of a wavelength of approximately 380 to 760 nm), thus failing to further adequately recognize the condition inside the surface layer, caries of a tooth, and tartar deposition condition.

On the other hand, patent document 2 discloses an apparatus capable of diagnosis of caries and the like by irradiating a tooth with an exciting light of wavelength of 405 nm and observing particular fluorescence emitted from lesion of the tooth through filter-fitted glasses having a transmission characteristic of 550 nm. Moreover, patent document 3 discloses an apparatus which irradiates a tooth with an exciting light of wavelength of 360 nm to 420 nm and, as is the case described above, observes particular fluorescence emitted from lesion of the tooth with glasses provided with a filter which absorbs or reflects the light with wavelength of 620 nm or below.

Moreover, patent document 3 discloses a technology of improving the background brightness by providing another light source for emitting a white light, in addition to a laser (exciting light) light source for inducing fluorescence radiation through irradiation of a tooth.
Patent document 1: Japanese Unexamined Patent Publication No. 1999-047092
Patent document 2: Japanese Unexamined Patent Publication No. 1984-137037
Patent document 3: Japanese Patent Publication No. 1994-73531

### DISCLOSURE OF THE INVENTION

### PROBLEM TO BE SOLVED BY THE INVENTION

The apparatus disclosed in the patent document 2 performs diagnosis by guiding light from a light source, such as a mercury lamp, to a probe via a fiber, irradiating the light from the probe to an affected part such as a tooth, and then observing condition of the affected part through the protective glasses provided with a filter. However, this apparatus requires the fiber and further another assistant who operates the probe, thus suffering from problems that the apparatus is large sized and has difficulty in achieving efficient diagnostic operation. Moreover, the apparatus disclosed in patent document 3 guides light from the light source via a ray guide, irradiates the light to a tooth, and observes a radiation light from the tooth with the glasses fitted with a filter. Also in this case, the apparatus requires the light guide, thus involving problems that the apparatus is large-sized and has difficulty in achieving efficient diagnostic operation.

Further, the apparatuses disclosed in patent documents 2 and 3 can determine whether a specific portion is a carious (decayed) part or a healthy part and determine distribution of the carious part only, and can obtain only detection information upon the irradiation of an exciting light of a specific wavelength. Thus, these apparatuses cannot simultaneously view both the carious part and a healthy normal tissue for the purpose of recognizing how and in which area of the healthy normal tissue the carious part is distributed, and also cannot be used as illumination for treatment. In another word, even through these apparatuses can detect only local carious part, they cannot obtain information which permits, by simultaneously viewing both the healthy normal tissue and the carious part, determination of the relative position and distribution condition of the caries in a full image of the healthy normal tissue of the tooth.

Specifically, the light intensity of the fluorescence emitted from the lesion through the irradiation of the exciting light is weak, and thus a fluorescence image generating this fluorescence is buried (masked) by the exciting light reflected from a subject portion to be observed or by a strong disturbance light, thus failing to sharply recognize the portion emitting the fluorescence; therefore, in patent documents 2 and 3 described above, observation is performed through the filters which cut off these exciting light or part of the disturbance light as much as possible. However, although cutting off the exciting light or part of the disturbance light by the filters in this manner permits the portion generating the fluorescence to be extracted and sharply viewed, but this also cuts off the minimum illumination light required for the observation. Thus, this causes, during observation in a dark place, such as in the oral cavity, the portion other than the lesion indicated by the fluorescence to appear dark and its outline to appear indistinct, and also may result in an unnatural image with lack of a blue color, depending on a filter to be used. Moreover, also in dermatology, in which acne bacteria is detected by using an exciting light in actual practice, the same problems arise.

Fluorescence obtained by an exciting light is fundamentally a very weak light. Thus, when exposed to illumination, such as a white light source, the fluorescence is buried in a reflection light of the illumination light and thus is hard to detect. Moreover, since an exciting light typically has a narrow band, in a case of an image of only this exciting light and a fluorescence or in a case of fluorescence with the exciting light cut, only the fluorescence portion is extracted, thus posing problems that healthy living tissue at the periphery of lesion cannot be clearly visually recognized, that a true color tone cannot be reproduced, and the like. In particular, most of the exciting light is cut when received, and thus an obtained image is just a fluorescence image, i.e., an indistinct image with the very dark background. From such an image, it is almost impossible to recognize the hue and texture of the subject, and it is possible just to visually recognize the indistinct outline.

Therefore, setting aside a case where diagnosis of only an affected part based on the image of an exciting light is involved, in a case where condition of healthy normal tissue at the periphery of the affected part is used for observation or explanation based on this image, the image has a color tone largely different from a true color tone, thus causing great discomfortable feeling. Thus, in the medical field, an affected part is, for better visual recognition, subjected to processing, such as image processing in which a fluorescence image and an illumination light image are individually used and photographed with a camera such as a CCD camera and then obtained images are superposed one on another to be observed on a monitor.

Patent document 3 describes that a white light, in addition to the exciting light (laser light), can be irradiated, but neither describes nor implies that the both lights are simultaneously irradiated to permit to visually recognize a clear fluorescence image of the lesion in a clear image of the healthy normal tissue, and also dose not disclose any technical idea that the relative luminance between the exciting light and the white light is appropriately adjusted to obtain a suitable image in accordance with a detection purpose. Further, a detection device for a carious tooth in this patent document leads a radioactive ray from an irradiation appliance by the ray guide, and there is no description that generators for an exciting light and an illumination light and a camera are integrated together, and thus the apparatus is inevitably large-sized.

In view of the problems described above, the present invention has been made, and is intended to provide a living body observing apparatus, an intraoral imaging apparatus, and a medical treatment appliance with simple configuration which are capable of sharply visually recognizing a fluorescence image of lesion in a sharp image of the periphery of an affected part and also which can be readily used by those people ranging from operators such as dentists and doctors to general users at home.

### MEANS FOR SOLVING THE PROBLEMS

A living body observing apparatus according to claim 1 is characterized by comprising irradiation means including an exciting light emission section emitting an exciting light for causing lesion to radiate fluorescence and an illumination light emission section emitting an illumination light for illuminating the periphery of the lesion, in which the irradiation means simultaneously irradiates the illumination light and the exciting light. It is desirable that the irradiation means described above, as with the present invention of claim 2, comprise light amount adjustment means for adjusting an irradiation amount of at least one of the illumination light and the exciting light. Further, as with the present invention of claim 8, it is desirable that the amount of light emitted from the exciting light emission section be larger than that of light emitted from the illumination light emission section. Such setting of large and small amounts of light emission is achieved by adjustment by the light amount adjustment means in each use in accordance with a purpose or by previous adjustment at a plant at shipment of products. Moreover, the amount of light emission can be changed appropriately by setting the number of light emission sections and volume adjustment.

The living body observing apparatus according to the present invention of claim 3 comprises means for selecting any one of a mode in which the exciting light emission section and the illumination light emission section are simultaneously caused to emit the light for the irradiation; a mode in which only the illumination light emission section is caused to emit the light for the irradiation; and a mode in which only the exciting light emission section is caused to emit the light for the irradiation. Adopted as the illumination light emission section of the irradiation means is, as with the present invention of claim 4, the one which emits one or more of a white light, a monochromatic light, an infrared light, and an ultraviolet light. Desirably adopted as the exciting light emission section of the irradiation means is the one which is, as with the present invention of claim 5, comprised of any one of an LED, a laser diode, a semiconductor laser, a solid state laser oscillator, and a laser oscillator, or the one which, as with the present invention of claim 6, is comprised of a combination of any one of a halogen lamp, a metal halide lamp, a xenon lamp, a krypton lamp, a mercury lamp, and a sodium lamp with an irradiation filter. It is desirable that, as with the present invention of claim 9, the irradiation means be detachably fitted to the body.

It is desirable that, as with the present invention of claim 7, the wavelength of the exciting light irradiated from the irradiation means be any one of 365 ± 30 nm, 400 ± 30 nm, and 470 ± 30 nm, although not limited thereto, and thus an exciting light in an infrared region can also be adopted. The living body observing apparatus according to the present invention of claim 10 is characterized in that the exciting light emission section and/or the illumination light emission section of the irradiation means emits a plurality of types of lights of mutually different wavelengths, and further comprises selection means for selecting one or more of the plurality of types of lights. The present invention of claim 11 is characterized by comprising irradiation driving means for causing the light selected in this manner to be emitted and irradiated.

The living body observing apparatus according to the present invention of claim 12 is characterized by further comprising, in addition to the above, imaging means for imaging a radiation light image formed of a fluorescence image from the lesion and/or an illumination light image from the periphery of the lesion. It is desirable that, as with the present invention of claim 13, the imaging means comprise a light receiving filter for cutting off the exciting light.

The present invention of claim 14 embodies the present invention described above in the intraoral imaging apparatus, characterized by comprising a main body including imaging means and the irradiation means according to any one of claims 1 to 11 which is provided in the main body. The present invention of claim 15 specifies a more desirable embodiment, characterized by comprising: a main body including imaging means; and irradiation means comprising an exciting light emission section emitting an exciting light for causing lesion to radiate fluorescence and an illumination light emission section emitting an illumination light for illuminating the periphery of the lesion, in which: the irradiation means and the imaging means are provided in the main body; the irradiation means simultaneously irradiates the illumination light and the exciting light; and in which the amount of the light emitted from the exciting light emission section is larger than the amount of the light emitted from the illumination light emission section.

It is desirable that, as with the present invention of claim 16, the imaging means also comprise a light receiving filter for cutting off the exciting light. It is desirable that, as with the present invention of claim 17, this light receiving filter be detachably fitted to the main body of the apparatus. It is also desirable that, as with the present invention of claim 18, the irradiation means comprise light amount adjustment means for adjusting the irradiation amount of at least one of the illumination light and the exciting light. As the imaging means, the one formed of a solid-state imaging device (CCD or MOS) is desirably adopted.

The present invention of claim 19 is characterized in that the irradiation means of the intraoral imaging apparatus comprises light amount adjustment means for simultaneously irradiating the illumination light and the exciting light and further switching irradiation amount balance between the illumination light and the exciting light.

The present invention of claim 20 refers to a medical treatment appliance which uses the living body observing apparatus or the intraoral imaging apparatus, characterized in that the living body observing apparatus or the intraoral imaging apparatus according to any one of claims 1 to 19 is integrally incorporated.

### EFFECTS OF THE INVENTION

According to the present invention of claim 1, an illumination light and an exciting light can be irradiated simultaneously, thus permitting simultaneously to visually recognizeg and observe a visible light image of the periphery of the lesion obtained through the irradiation of the illumination light and a fluorescence image of the lesion obtained through the irradiation of the exciting light. Therefore, the position of the lesion relative to healthy normal living tissue at the periphery of the lesion and the degree of the lesion can be adequately recognized, thus providing image information of high diagnostic and clinical values. Especially in application to dental caries diagnosis, the irradiation of the illumination light permits the gum and the outline of a tooth around the caries to be clearly visually recognized, and also the irradiation of the exciting light permits a fluorescence image specific to the caries to be clearly recognized, thus largely contributing to an improvement in the accuracy in subsequent treatment.

In addition to the above, as with the present invention of claim 2, if the irradiation means includes light amount adjustment means for adjusting an irradiation amount of at least one of the illumination light and the exciting light, for example, the irradiation amount of the exciting light can be so adjusted as to be relatively larger than that of the illumination light in order to stress the fluorescence image of the lesion, while the irradiation amount of the illumination light can be so adjusted as to be relatively larger than that of the exciting light in order to stress the visible light image of healthy normal tissue at the periphery of the lesion and observe the image in the state close to an image with a natural color tone. This permits easily obtaining a useful image in accordance with application and diagnostic purposes. Then, as with the present invention of claim 3, if selection can be made from among three types of modes, flexible measures can be taken smoothly in accordance with application and diagnostic purposes, such as simultaneously obtaining an image of healthy normal tissue at the periphery of the lesion and an image of the lesion, obtaining only the image of the lesion, obtaining only the image of the healthy normal tissue at the periphery of the lesion, and the like.

As with the present invention of claim 4, if an illumination light emission section emits one or more of an white light, a monochromatic light, an infrared light, and an ultraviolet light, the image of healthy normal tissue at the periphery of the lesion results in an image having a natural color tone with favorable texture especially when the white light is emitted, whereas, when the visible monochromatic light in red, green, yellow, or the like is emitted, the image having a natural color tone with favorable texture as described above cannot be obtained but the healthy normal tissue at the periphery of the lesion can appear in various color patterns as with a case, for example, where a little more redness is desired, where only a yellow color component is desired to be added, or the like, thus permitting obtaining an image of a high practical value. Further, when the infrared light or the ultraviolet light is used as the illumination light, a reflection light image thereof does not represent a visible light and thus cannot be visually recognized directly, but observing it through imaging means such as a CCD covering a frequency band up to the infrared light or the ultraviolet light permits obtaining an image of a high clinical value due to a characteristic (for example, large permeability) possessed by the infrared light or the ultraviolet light.

As with the present invention of claim 5, if the exciting light emission section of the irradiation means is comprised of any one of an LED, a laser diode, a semiconductor laser, a solid state laser oscillator, and a laser oscillator, the width of wavelength of the exciting light irradiated from the irradiation means is limited, thus permitting construction without the use of an irradiation optical filter. Moreover, the use of these general-purpose products permits low-cost construction. Adoption of the LED, the laser diode, the semiconductor laser, or the solid state laser oscillator in particular permits downsizing and weight saving of the equipment.

According to the present invention of claim 6, the exciting light emission section of the irradiation means described above is comprised of a combination of any one of a halogen lamp, a metal halide lamp, a xenon lamp, a krypton lamp, a mercury lamp, and a sodium lamp, i.e., white light or light close to white light having a broad wavelength, with an irradiation filter. Thus, to observe the fluorescence image of the lesion, the irradiation filter is fitted to extract only an exciting light component for irradiation, while these irradiation means can also be used for illumination when irradiation is performed with the irradiation optical filter removed.

Irradiation for caries, tartar, plaque, or the like of a tooth with the exciting light having a wavelength characteristic as the present invention of claim 7 causes fluorescence radiation from the caries, tartar, or plaque in a visible region, which is extremely effective in observing the caries, tartar, plaque, or the like. Moreover, as an emitter for emitting such an exciting light, a commercially available low-price product is applicable, which permits achieving cost reduction of the device product.

According to the present invention of claim 8, the amount of light emitted from the exciting light emission section is larger than the amount of light emitted from the illumination light emission section. Thus, the fluorescence image of the lesion obtained through the irradiation of the exciting light can be clearly visually recognized since masking of this image by the irradiation of the illumination light can be reduced. Moreover, the illumination light permits the image of the periphery of the lesion to be clearly recognized. Therefore, simultaneously visually recognizing the both images provides image information of an extremely high practical value.

According to the present invention of claim 9, the irradiation means is fitted detachably. Thus, fitting appropriate irradiation means suitable for an observation purpose permits various observations and also provides great convenience in maintenance of the device, in its attachment to a therapeutic apparatus, and the like.

According to the present invention of claim 10, the exciting light emission section and/or the illumination light emission section of the irradiation means emits a plurality of types of lights of mutually different wavelengths, and further comprises selection means for selecting one or more of the plurality of types of lights. Thus, appropriate selection by this selection means permits emitting and irradiating light of a wavelength in accordance with an observation purpose. In addition, as with the present invention of claim 11, if the selected light is caused to be emitted and irradiated by irradiation driving means, emission control can be performed based on a predetermined sequence. According to the present invention of claim 11, the irradiation driving means for causing the selected light to be emitted and irradiated is further included. Thus, for example, irradiating each of the selected lights in a time-shared manner causes a superposed image to be viewed by use of a residual image phenomenon without using an imaging device.

According to the present invention of claim 12, imaging means for imaging a radiation light image formed of a fluorescence image from the lesion and/or an image of the illumination light from the periphery of the lesion is included. Thus, the fluorescence image generated from the lesion based on the irradiation of the exciting light from the irradiation means is received by the imaging means, and/or an image of a reflection light reflected from the periphery of the lesion based on the irradiation of the illumination light is received by the imaging means, and an optical image converted into an electrical signal can be displayed and observed on a TV monitor or the like, both the image of healthy normal tissue and the image of the lesion can be viewed at least simultaneously, and further the fluorescence image alone or only the illumination light image can be visually recognized. This therefore permits obtaining image information of a high clinical value and also permits making highly accurate diagnosis.

Adopting a solid state imaging device (CCD or MOS) as the imaging means permits quickly obtaining favorable imaged image information at low costs. Moreover, this can be provided as a compact, easy-to-handle, and convenient type, and further can be commercialized as a compact, low-cost, and safe type. Thus, the specifications with limited functions are optimum for home use. The imaging means with a wide spectral characteristic capable of photographing light of a wavelength of, for example, 300 to 800 nm can photograph a portion where fluorescence is emitted, and also can, needless to say, photograph lights widely from the ultraviolet light to the infrared light including the visible lights. When detection of special fluorescence is required, the one with a wide spectral characteristic for a wavelength range wider than the range described above can be of course selected.

As with the present invention of claim 13, if the imaging means includes a light receiving filter for cutting off the exciting light, the exciting light reflected at the periphery of the lesion does not enter the imaging means, so that the fluorescence image from the lesion is not masked by the exciting light and thus is sharply visually recognized. It is needless to say that the light receiving filter here is, in another word, provided with a cutoff characteristic for cutting off the exciting light emitted from the exciting light emission section and thus transmits only light of a particular wavelength (here, transmits light which is closer to the visible light side than the exciting light described above).

According to the present invention of claims 14 and 15, if the exciting light from the exciting light emission section of the irradiation means and the illumination light from the illumination light emission section are simultaneously irradiated with the main body tip side portion inserted in the oral cavity, the fluorescence generated from the lesion and the reflection light from the healthy normal tissue at the periphery of the lesion can be received by the imaging means to obtain predetermined image information. The obtained image information relates to an image including the image of healthy normal tissue at the periphery of the lesion obtained by the reflection light of the illumination light and the image of the lesion obtained by the fluorescence, which are simultaneously and clearly visually recognized, and this image is an image of a high clinical value which permits adequately recognizing the relative position and degree of the lesion. If, upon the simultaneous irradiation, the amount of light emitted from the exciting light emission section is larger than the amount of light emitted from the illumination light emission section, the fluorescence image is less masked by the reflection light, so that the image of the lesion is more clearly visually recognized in the image of healthy normal tissue at the periphery of the lesion. Then, as described above, the optical image converted into an electrical signal by the imaging means can be displayed and observed on the TV monitor or the like, thus permitting diagnosis to be made while communicating with a patient or permitting the image to be stored as useful carte information.

Also in this case, as with the present invention of claim 16, if the imaging means includes a light receiving filter for cutting off the exciting light, the exciting light reflected at the periphery of the lesion dose not enter the imaging means, the fluorescence image from the lesion is not masked by the exciting light and thus is sharply visually recognized. As with the present invention of claim 17, if this light receiving filter is detachably fitted to the main body of the apparatus, a plurality of light receiving filters having different transmission (cutoff) characteristics are appropriately and selectively fitted in accordance with observation and diagnostic purposes, thereby permitting obtaining information on various observations and diagnosis.

As with the present invention of claim 18, if, also in the intraoral imaging apparatus, the irradiation means includes light amount adjustment means for adjusting an irradiation amount of at least one of the illumination light and the exciting light, for example, the irradiation amount of the exciting light can be so adjusted as to be relatively larger than that of the illumination light in order to stress the fluorescence image of the lesion, while the irradiation amount of the illumination light can be so adjusted as to be relatively larger than that of the exciting light in order to stress the visible light image of healthy normal tissue at the periphery of the lesion and observe the image in a state close to an image with a natural color tone. This permits easily obtaining a useful image in accordance with application and diagnostic purposes.

As with the present invention of claim 19, the irradiation means in the intraoral imaging apparatus includes light amount adjustment means for, while simultaneously irradiating the illumination light and the exciting light, switching irradiation amount balance between the illumination light and the exciting light. Thus, selection can be made from among: observation in which the lesion and the healthy normal tissue at the periphery of the lesion are simultaneously visually recognized with a natural color tone; observation focused on the lesion; and observation focused on the normal tissue at the periphery of the lesion. This permits simultaneous observation of the healthy normal tissue at the periphery of the lesion and the lesion with optimum light amount balance.

As with the present invention of claim 20, if a medical treatment appliance is constructed by integrally incorporating the living body observing apparatus or intraoral imaging apparatus described above, intended treatment can be adequately provided based on observation information or imaging information. Examples of such a medical treatment appliance include: for dental treatment, an air turbine handpiece, a micromotor handpiece, a scaler handpiece, a photo-polymerization irradiation apparatus, a tooth surface cleaning handpiece, a dental mirror, and the like; and for general medical treatment, a skin diagnostic examination appliance.

### BRIEF DESCRIPTION OF THE DRAWINGS

[FIG. 1] FIG. 1 is a plane view showing one example of an intraoral imaging apparatus of the present invention.
[FIG. 2] FIG. 2 is a longitudinal sectional view of the same intraoral imaging apparatus along the longitudinal direction thereof.
[FIG. 3] FIG. 3 is an enlarged bottom view of the main body tip side portion of the same apparatus.
[FIG. 4] FIG. 4 is a longitudinal sectional view, taken on line X-X of FIG. 3.
[FIG. 5] FIG. 5 is an operation diagram showing the condition of intraoral diagnosis performed by the intraoral imaging apparatus.
[FIG. 6] FIG. 6 is a diagram showing one example of a photographed image of teeth in the oral cavity.
[FIG. 7] FIG. 7 is a graph contrasting the radiation intensity, provided by an exciting light irradiation, between a carious (decayed) (decayed) tooth and a healthy tooth.
[FIG. 8] FIG. 8 is a graph showing a relative fluorescence intensity, provided by the exciting light irradiation, between a carious (decayed) tooth and a healthy tooth.
[FIG. 9] FIG. 9 is a graph showing the relationship between wavelength distribution of the exciting light and transmission characteristics of a light receiving filter section.
[FIG. 10] FIG. 10 is a partially cut-away longitudinal elevation of a second embodiment of the intraoral imaging apparatus.
[FIG. 11] FIG. 11 is a partially cut-away longitudinal elevation of a third embodiment of the intraoral imaging apparatus.
[FIG. 12] FIG. 12 is a partially cut-away longitudinal elevation of a fourth embodiment of the intraoral imaging apparatus.
[FIG. 13] FIGS. 13A to 13C are diagrams showing a first embodiment of a diagnostic irradiation head device, with 13A as a conceptual diagram with the device in use, 13B as an enlarged sectional view of irradiation means, and 13C as the same diagram of a modified example.
[FIG. 14] FIGS. 14A and 14B are diagrams showing a second embodiment of the diagnostic irradiation head device, with 14A as a conceptual diagram with the device in use and 14B as a partially cut-away enlarged perspective view of the irradiation means.
[FIG. 15] FIGS. 15A and 15B are diagrams showing a third embodiment of the diagnostic irradiation head device, with 15A showing an example in which the position of a plurality of emission sections are changed by being slid to selectively achieve irradiation and 15B showing an example in which the position of the same emission sections are changed by being rotated to selectively achieve irradiation.
[FIG. 16] FIGS. 16A and 16B are diagrams showing a fourth embodiment of the diagnostic irradiation head device, with 16A as a conceptual diagram with the device in use and 16B as a diagram showing detachment mechanism.
[FIG. 17] FIGS. 17A and 17B are diagrams showing a fifth embodiment of the diagnostic irradiation head device, with 17A as a diagram showing mechanism of detaching an orbit protective member and 17B as a conceptual diagram with the member in use.
[FIG. 18] FIGS. 18A and 18B are diagrams showing a sixth embodiment of the diagnostic irradiation head device, with 18A as a perspective view of a binocular telescope and 18B as a diagram showing mechanism of detaching the binocular telescope.
[FIG. 19] FIG. 19 is a diagram showing a seventh embodiment of the diagnostic irradiation head device.
[FIG. 20] FIG. 20 is a diagram showing an eighth embodiment of the diagnostic irradiation head device.
[FIG. 21] FIG. 21 is a diagram showing an embodiment of a dental mirror.
[FIG. 22] FIG. 22 is a longitudinal sectional elevation of an example in which a living body observing apparatus of the present invention is configured as a dental mirror type.
[FIG. 23] FIG. 23 is a plane view of the same example.
[FIG. 24] FIG. 24 is an overall perspective view of the same example.
[FIG. 25] FIGS. 25A and 25B are diagrams explaining the schematic configuration of an example and a modified example in which the living body observing apparatus of the present invention is incorporated into a tooth cutting handpiece.
[FIG. 26] FIGS. 26A, 26B, and 26C are diagrams showing a specific example of the head part of the handpiece in the same embodiment.
[FIG. 27] FIG. 27 is a diagram showing a modified example of the same modified example.
[FIG. 28] FIG. 28A is a diagram showing an example of mechanism of detaching the irradiation means at the head of the handpiece in the same embodiment, and FIG. 28B is a diagram showing the irradiation means in the detached state.
[FIG. 29] FIGS. 29A and 29B are diagrams showing further modified example of FIG. 25B.
[FIG. 30] FIG. 30 is a diagram showing another embodiment of the tooth cutting handpiece.
[FIG. 31] FIGS. 31A and 31B are diagrams explaining the schematic configuration in an example and a modified example, respectively, in which the living body observing apparatus of the present invention is incorporated into a dental scaler handpiece.
[FIG. 32] FIGS. 32A and 32B are diagrams showing the same modified example.
[FIG. 33] FIG. 33 is a diagram describing the schematic configuration in an example in which the living body observing apparatus of the present invention is incorporated into a dental light probe.
[FIG. 34] FIG. 34 is a diagram explaining the schematic configuration in an example in which the living body observing apparatus of the present invention is incorporated into a dental photo-polymerizer.
[FIG. 35] FIGS. 35A and 35B are diagrams explaining the schematic configuration in an example in which the living body observing apparatus of the present invention is incorporated into a laser therapeutic apparatus, with 35A as an elevation and 35B as a lateral sectional view of a modified example.
[FIG. 36] FIG. 36 shows an electrical circuit for achieving light amount balance between the exciting light and the illumination light by adjustment of a variable resistor.
[FIG. 37] FIG. 37 shows an electrical circuit capable of switching an initial setting of the light amount balance between the exciting light and the illumination light at shipment from the plant and an adjustment of the variable resistor arbitrarily performed by the user after the shipment.
[FIG. 38] FIG. 38 is a control block diagram showing one example of an irradiation light source switching structure.
[FIG. 39] FIG. 39 is a circuit diagram showing another example of the light amount adjustment means.

### REFERENCE NUMERALS

2: Irradiation means
2a: Exciting light emission section
2b: Illumination light emission section
4, 40: Imaging means
5, 39, 41, 43, 49, 55, 60: Light receiving filter
66: Irradiation filter
97, 98, 101, 102: Variable resistor for light amount adjustment (light amount adjustment means)
A, A1 to A3: Intraoral imaging apparatus
F, F1 to F4: Dental handpiece (medical treatment appliance)
G, G1 to G2: Dental scaler handpiece (medical treatment appliance)
H: Dental light probe (medical treatment appliance)
I: Dental photo-polymerizer (medical treatment appliance)
J: Dental laser treatment device (medical treatment appliance)

### BEST MODE FOR CARRYING OUT THE INVENTION

Hereinafter, the preferred embodiments of the present invention will be described, with reference to the accompanying drawings.

### FIRST EMBODIMENT

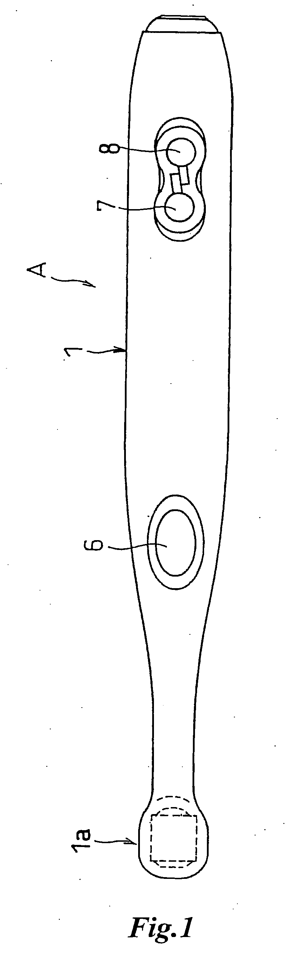

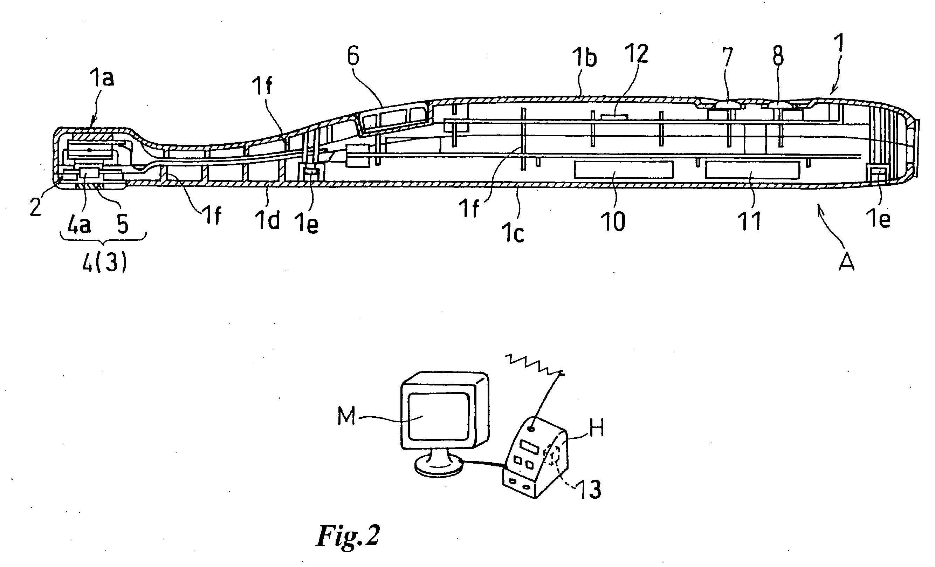

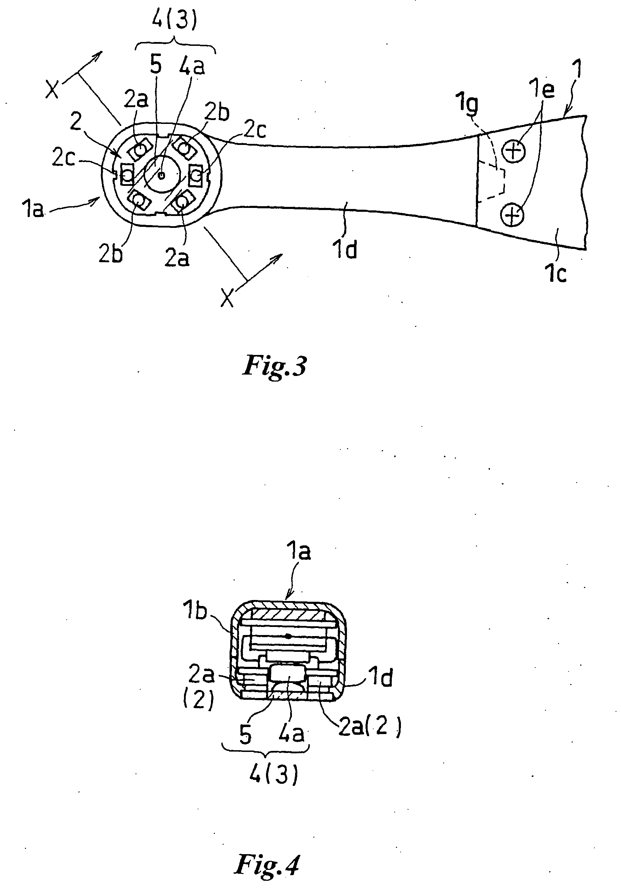

FIG. 1 is a plane view showing an example of a living body observing apparatus of the present invention embodied as an intraoral imaging apparatus. FIG. 2 is a longitudinal sectional view of a main body of the same imaging apparatus along the longitudinal direction thereof. FIG. 3 is an enlarged bottom view of the main body tip side portion of the same apparatus. FIG. 4 is a longitudinal sectional view, taken on line X-X of FIG. 3. The intraoral imaging apparatus A in the figures includes, at a head part 1a of the main body (casing) 1 thereof in the form of a dental handpiece supportable by hands and fingers, irradiation means 2 and imaging means 4 as the observation section 3. This intraoral imaging apparatus A is suitable for use mainly in diagnosis of caries of a tooth, a missing part, lesion, the degree of adhesion of tartar, plaque, and a biofilm, and the like in the oral cavity, and can be further designed as a cordless-type which is capable of wirelessly transmitting a signal to a control box H (see FIG. 2) and extracting a photographed image by printing it out. In addition, zooming mechanism of zooming in and out or an autofocus function can also be provided in the intraoral imaging apparatus A.

The main body casing 1 is composed of an upper case 1b, a lower case 1c, and the tip end side case 1d formed of synthetic resin, in which the lower case 1c is fixed to the upper case 1b with four screws 1e. The main body casing 1 has a relatively thick trunk which can be supported by hands and fingers and which is once so tapered increasingly toward the tip end side, thus forming the head part 1a of a shape expanding upward and also to the left and right. The imaging means 4 is composed of a solid state imaging device 4a, such as a CCD or a MOS, and a light receiving filter 5 for directing light of a particular wavelength to the solid state imaging device 4a, and is included in the tip end side case 1d which is detachably fitted to the upper and lower cases 1b and 1c. Numeral 1f denotes reinforcing ribs integrally formed inside the cases 1b to 1d.

Since the light receiving filter 5 is formed in the tip end side case 1d detachable from the main body casing 1, the tip end side case 1d can be replaced with a tip end side case 1d having a different light receiving filter 5 with a different wavelength characteristic. In FIG. 4, the irradiation means 2 is installed in the upper case 1b together with the imaging means 4, but may be alternatively fitted in the tip end side case 1d so that the entire tip end side case 1d together with the light receiving filter 5 can be replaced. In this condition, an electrical contact for supplying electrical power to the irradiation means 2 may be so constructed as to be separable.

On the top surface of the main body casing 1, a photographing switch 6, a light source selection switch 7, an image selection switch (selection switch for selecting an image to be displayed on the monitor from among photographed images) 8 are formed. Provided in the main body casing 1 are: a power source 10, such as a secondary battery, for driving the irradiation means 2, the imaging means 4, and the like; a radio transmitter 11 for transmitting information imaged by the imaging means 4 to the control box H; and a microcomputer 12. That is, this intraoral imaging apparatus A is constructed as a cordless type which permits easy operation without routing lead wires. If the intraoral imaging apparatus A is not a cordless type, the photographing switch 6 may be provided in a place, other than the main body casing 1, such as the control box H or a foot pedal (omitted from illustration), which is connected to the intraoral imaging apparatus A via the lead wire. To the control box H, a monitor M for displaying an intraorally photographed image is connected.

Arranged in the head part 1a are: as shown in FIGS. 3 and 4, the imaging means 4, the irradiation means 2, and the light receiving filter 5 constituting the imaging means 4. The imaging means 4 is composed of the solid state imaging device 4a, such as a CCD or a MOS, and the light receiving filter 5 as described above. The imaging means 4, when a subject portion to be observed such as a tooth is irradiated with an irradiation light from the irradiation means 2, receives light radiated from the subject portion to be observed and photographs a predetermined diagnostic image. The imaging means 4 is arranged at the central position of the head part 1a as viewed vertically.

The irradiation means 2 is composed of, as shown in FIG. 3, two each of three types of LEDs (light-emitting diodes): an exciting light emitting LED 2a as an exciting light emission section and a white light LED 2b and an infrared light LED 2c as illumination light emission sections, i.e., six LEDs in total, which are arranged substantially equiangularly around the optical axis of the solid state imaging device 4a so as to be rotationally symmetrical. This therefore permits construction that light from the irradiation means 2 can be directly irradiated to a tooth. Each two of the LEDs 2a, 2b, and 2c is arranged 180 degrees apart circumferentially around the solid state imaging device 4a so as to face each other. If the LED for an illumination light and the LED for an exciting light provide the same amount of lights and exhibit the same or similar intensity distribution, the number of LEDs for an illumination light is adapted to be smaller than the number of LEDs for an exciting light or the light intensity of the LED for an illumination light is adapted to be weaker than that of an exciting light to thereby reduce an output of the white light LED 2b. This reduces masking of a fluorescence image obtained through the irradiation of the exciting light by a reflected illumination light, thus permitting the fluorescence image to be clearly visually recognized. Simultaneous irradiation of the white light LED 2b and the exciting light LED 2a permits simultaneous observation of both lesion such as caries and the healthy normal tissue at the periphery of the lesion.

The irradiation means 2 may include only the exciting light emitting LEDs 2a and white light LEDs 2b described above, and they may be constructed in appropriate combination with the infrared light LEDs 2c described above or an ultraviolet light LED. The plurality of emission sections can be selectively caused to emit light through operation of the light source selection switch 7 shown in FIG. 1. Adopted as the irradiation means 2, other than LEDs, can be any one of a laser oscillator (a semiconductor laser such as an He-Ne laser, a krypton laser, or a dye laser; a laser diode, a solid state laser oscillator, or a laser oscillator), a halogen lamp, a krypton lamp, a mercury lamp, a sodium lamp, a xenon lamp, and a metal halide lamp. The use of an LED, a laser diode, or the like permits achieving downsizing and weight saving.

To use the halogen lamp, the krypton lamp, the mercury lamp, the sodium lamp, the xenon lamp, or the metal halide lamp as the exciting light emission section of the irradiation means, an irradiation filter may be placed in front of the lamp to extract and irradiate only light of a wavelength of an exciting light component. Since light irradiated from the lamp light source described above is a white light or light close to white light having a broad wavelength, providing a portion without the irradiation filter permits this portion to serve as an emission section for irradiation, it may be an LED or a laser oscillator of a wavelength switching type. To use the laser oscillator, light from the laser oscillation section located in the main body or separately installed needs to be guided to the head irradiation port of the head part 1a by using an adequate ray guide.

A desirable illumination light emitting LED is the one having not only a range including infrared, near-infrared, ultraviolet, near-ultraviolet in addition to a white light, but also a range including red, Mars yellow, purple, blue, and green in a visible light range. A desirable laser transmitter for an illumination light is also the one having not only a range including infrared, near-infrared, ultraviolet, and near-ultraviolet, but also a range including red, Mars yellow, purple, blue, and green in a visible light range. Moreover, desirably adopted as an exciting light emitting LED is the one which emits light of a central wavelength of 365 nm, 405 nm, or 470 nm. These LEDs are available at low price from those generally commercially available. Adopted as the wavelength of a laser light emitting an exciting light is 635 nm or 780 nm as a central wavelength.

The light receiving filter 5, as shown in FIGS. 2 to 4, is fitted by being detachably fitted in the tip end side case 1d for replacement, and is formed into a substantially circular plate-like shape in a size so dimensioned as to cover the lower side of the solid state imaging device 4a. The tip end side case 1d itself is also detachable from the lower part of the main body 1 for replacement. This light receiving filter 5 permits only light of a wavelength in a particular range to pass therethrough toward the light receiving section of the solid state imaging device 4a. The periphery corresponding to the irradiation means 2 may be structured to permit light of the irradiation means 2 to directly pass therethrough, or this periphery may be provided as an irradiation filter which permits, of lights from the irradiation means 2, only light of a wavelength in a particular range, to pass therethrough to irradiate the light to a subject to be diagnosed or may be provided as a composite filter provided with functions of both a light receiving filter and an irradiation filter. Moreover, the light receiving filter, the irradiation light filter, and a lens may be used in combination, or these filters and the lens may be integrally constructed. Furthermore, the surface of these lenses may be provided with a coating having a filter function for use.

The light receiving filter 5, when an exciting light is irradiated from the exciting light emitting LED 2a of the irradiation means 2 and also an illumination light is irradiated from the white LED 2b to cause particular fluorescence (visible light) to radiate from lesion and also cause a reflection light of the illumination light to radiate from the periphery of the lesion whereby a fluorescence image and a reflection light image are observed and photographed by the imaging means 4, transmits the fluorescence image and the reflection light image obtained by the illumination light described above. Thus, adopted as the light receiving filter 5 is the one having such a transmission (cutoff) characteristic as to cut off an exciting light reflected from the periphery of the lesion.

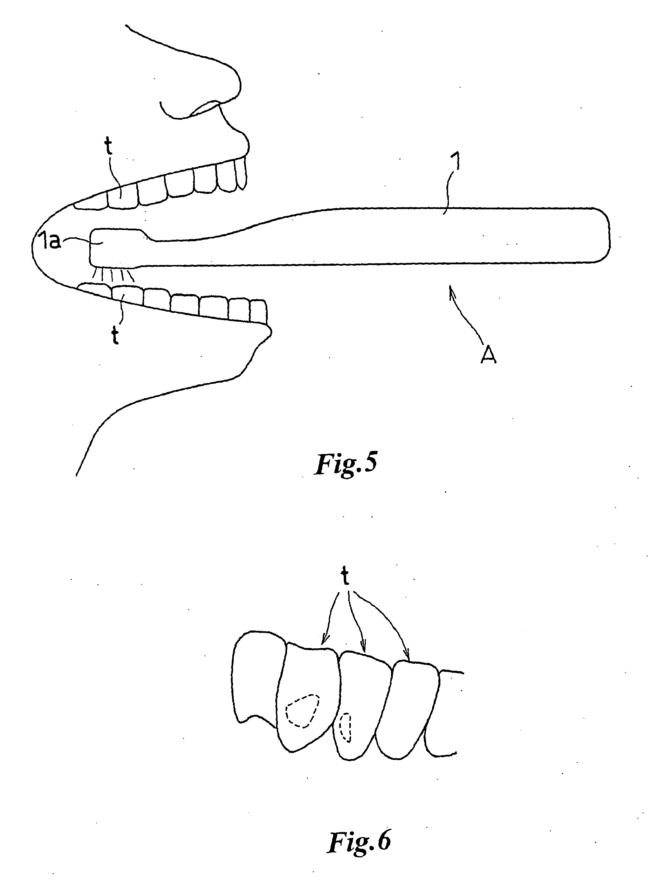

FIG. 5 is a diagram showing the intraoral imaging apparatus A described above in use. As shown in the figure, this intraoral imaging apparatus A permits diagnosis of the tooth t as a subject to be observed and diagnosed with the head part 1a inserted in the oral cavity, and permits diagnosis while representing an image imaged by the imaging means 4 on the screen of the monitor M connected to the control box H (see FIG. 2 for the both). The intraoral imaging apparatus A, if there is a region of interest, can store a still image of the region into the main body casing 1 or a memory 13 of the control box H through operation of the photographing switch 6 while photographing this area, and, when needed, can print out the image by a printer connected to the control box H (omitted from illustration). This intraoral imaging apparatus A can irradiate an irradiation light from the irradiation means 2 directly to the tooth t.

A tooth with tartar, plaque, or a carious portion (lesion) is irradiated with an exciting light of a central wavelength of 405 nm by the exciting light emitting LED 2a of the irradiation means 2 and also irradiated with an illumination light by the white LED 2b, and then is photographed with the light receiving filter 5, which transmits only light closer to the visible light side than the wavelength range of this exciting light, fitted to the light receiving section of the imaging means 4. As a result, these lesions in the diagnostic image are visually recognized as orange to Mars yellow. FIG. 6 shows a printed image of the tooth t photographed by the imaging means 4 of the intraoral imaging apparatus A. Through the irradiation of the exciting light, fluorescence as described above is radiated from the lesion, and this fluorescence image is transmitted through the light receiving filter 5 and then formed on the solid state imaging device 4a. Moreover, an image of the periphery of the lesion (outline of the tooth t) obtained by the illumination light simultaneously irradiated is also transmitted through the light receiving filter 5, and then formed on the solid state imaging device 4a. In FIG. 6, parts marked by dashed lines correspond to the lesions.

Most of the exciting light reflected on a region other than the lesion is cut off (reflected or absorbed) by the light receiving filter 5; thus, the fluorescence image described above is not masked by these exciting lights and thus is clearly visually reconginzed. In addition, the image of the tooth t and the image of healthy normal tissue at the periphery, such as the gum, of the lesion, which is irradiated with the illumination light, can also be clearly formed on the solid state imaging device 4a. Therefore, in the image displayed on the monitor M and the printed image of FIG. 6, the images of the tooth and the healthy normal tissue at the periphery such as the gum of the lesion can also be clearly visually recognized with natural color tones, and, in the image, the image of the lesion (portion emitting fluorescence) appearing orange to Mars yellow is clearly visually recognized.

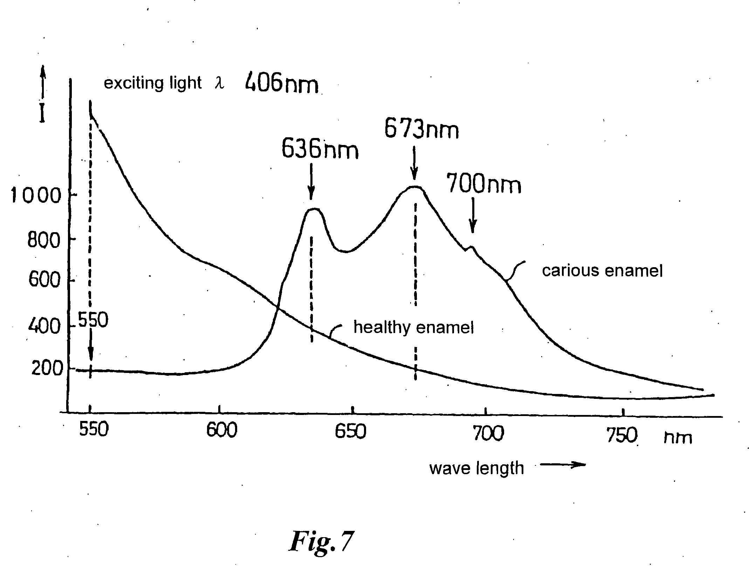

FIG. 7 shows spectrum distribution of light (fluorescence) radiated from teeth which were irradiated with an exciting light of a wavelength of 406 nm. In the figure, the healthy tooth shows a tendency that the radiation intensity I gradually decreases with an increase in the wavelength of the fluorescence, while the carious (decayed) tooth shows a fluorescence spectrum with peaks of radiation intensity with respect to the wavelength at three points (636 nm, 673 nm, and 700 nm). In addition to the above, the experiment has confirmed that fluorescence in orange to Mars yellow is also emitted. Therefore, if, in the subject portion to be observed, a portion emitting such fluorescence is visually recognized, this portion can be adequately determined to be a carious portion.

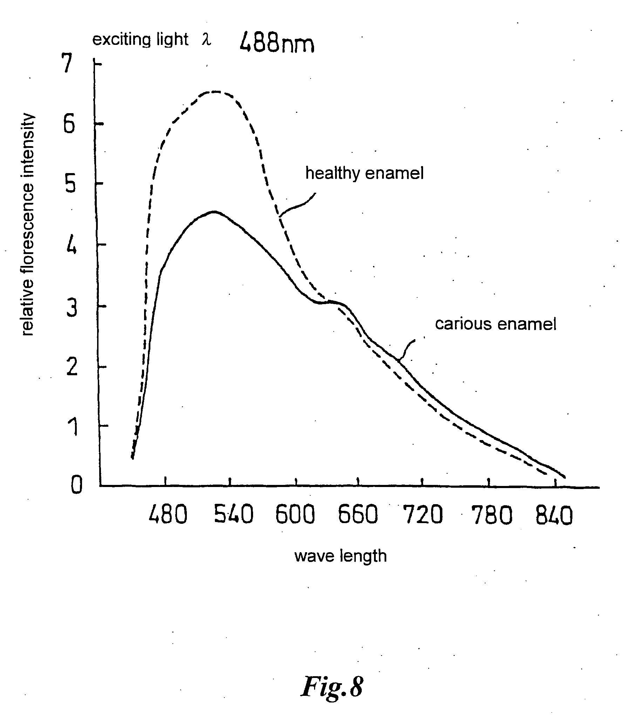

FIG. 8 is a graph (graph contrasting fluorescence intensity) contrasting lights radiated from a healthy tooth (healthy enamel) and a carious tooth (carious enamel) which are irradiated with an exciting light of a wavelength of 488 nm. As can be understood from this graph, between the healthy tooth and the carious tooth, different wavelengths of an exciting light to be irradiated typically results in different intensities for each wavelength of generated fluorescence. For a tooth, as in FIG. 7, it is understood that remarkable difference is seen at 600 nm or higher when an exciting light of a wavelength of 406 nm is irradiated. Moreover, fluorescence generated from caries or the like through irradiation of an exciting light is typically a very weak light; thus, when an affected part such as caries is simultaneously irradiated with an illumination light, the fluorescence is buried in the illumination light and thus is hard to be visually recognized. Further, to visually recognize a very weak fluorescence, it needs to be seen in a dark place, but the fluorescence cannot be seen as clearly as entire tissue of the subject portion to be observed and diagnosed can be seen under a normal natural light or a white light; thus, the periphery of the affected part or the background tissue cannot be seen in the complete absence of an illumination light. Thus, it is required to permit viewing while locating the affected part relative to the periphery thereof.

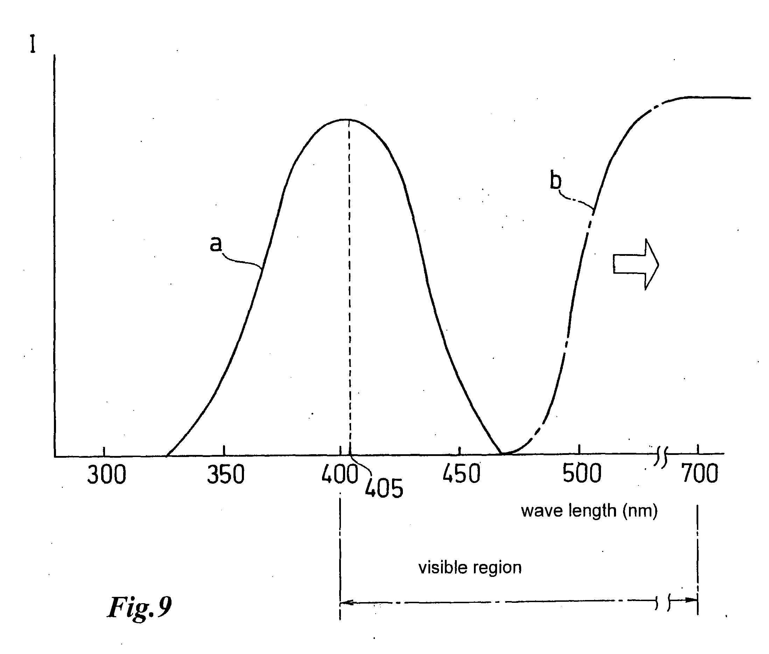

FIG. 9 is a diagram showing the relationship between a wavelength distribution characteristic of an exciting light irradiated from the irradiation means 2 and a transmission characteristic of the light receiving filter 5. In the figure, symbol "a" denotes the wavelength distribution characteristic of an exciting light of a central wavelength of 405 nm, and symbol "b" denotes the transmission (cutoff) characteristic curve line of the light receiving filter 5 used in correspondence therewith. Specifically, this figure indicates that the light receiving filter 5 transmits light on the longer wavelength side of this transmission characteristics curve line b, that is, the center side of a visible light (band indicated by a hollow arrow). An LED or the like as an emission section of exciting light is designed to emit light of a single wavelength, but, in practice, it inevitably has a wavelength distribution characteristic in some range, as shown in the figure. Therefore, if the light receiving filter 5 is so designed as to be capable of transmitting part of light on the longer wavelength side of the central wavelength of the exciting light, the aforementioned part of the exciting light can be used as an illumination light for the periphery of the lesion. Especially when a white light is used as an illumination light and the light receiving filter described above is used which transmits only a wavelength of 500 nm or higher, this results in a color tone with a blue light of near 450nm removed. However, if a blue exciting light is irradiated simultaneously with an illumination light, part of the removed blue component makes a contribution as a part of the illumination light, which can avoid the color tone from becoming unnatural.

Thus, when the tooth t is irradiated with the exciting light of the wavelength described above, and simultaneously therewith, irradiated with the illumination light, the lesion of the tooth t radiates fluorescences of wavelengths of 636 nm, 673 nm, and 700 nm, respectively, as shown in FIG. 7, and these fluorescences are transmitted through the light receiving filter 5 and can be visually recognized as images. The exciting light which is also reflected on the portion other than the lesion of the tooth t, as can be understood from the transmission characteristics curve line b of FIG. 9, is cut off by the light receiving filter 5, although, of the lights reflected from the periphery of the lesion through the irradiation of the illumination light, the light on the longer wavelength side of the transmission characteristics curve line b is transmitted. As a result, the image of healthy normal tissue at the periphery of the lesion of the tooth t is visually recognized. Therefore, a fluorescence image of the lesion extracted without being masked by the reflected exciting light and the image of the healthy normal tissue at the periphery of the lesion of the tooth t can be visually recognized simultaneously, thus obtaining image information of an extremely high diagnostic value. Moreover, providing the light receiving filter 5 in front of the solid state imaging device 4a permits the fluorescence image of the lesion and the image of the healthy normal tissue at the periphery of the lesion to be simultaneously formed and observed on the solid state imaging device 4a.

The intraoral imaging apparatus A in the illustrated example, as described above, is provided with: as the irradiation means 2, the exciting light emitting LEDs 2a and the white LEDs 2b for illumination light emission, and also the infrared light LEDs 2c. In such a construction, for example, the illumination light and the exciting light can be emitted simultaneously and observed as the monitor images by using the imaging device, and independently therefrom, selection can be made through operation of the light source selection switch 7 to thereby light up only the white LED 2b, which then can be used as an illumination light for observation of condition of the subject portion to be observed and the periphery thereof. Also, when only an illumination light is in use, the operator himself or herself can perform visual recognition and observation without driving the imaging means 4 (with the image selection switch 8 turned off). Moreover, taking advantage of its strong permeability characteristic, the infrared light LED 2c is used for observation of a deep part of a tooth, such as a crack. In this case, the operator himself or herself can observe a reflected infrared light image, but also by using the light receiving filter 5 transmitting only an infrared light, the extracted infrared light image can be sharply visually recognized by the imaging means 4, although the healthy normal tissue cannot be visually recognized. Even when only the white LEDs 2b is lit up and this is used as an illumination light to obtain the imaged image of condition of the subject portion to be observed and the periphery thereof, a filter transmitting light of a wavelength of approximately 450 nm or higher does not have much influence in the obtained image, and thus can be satisfactorily used even when left loaded.

### SECOND EMBODIMENT

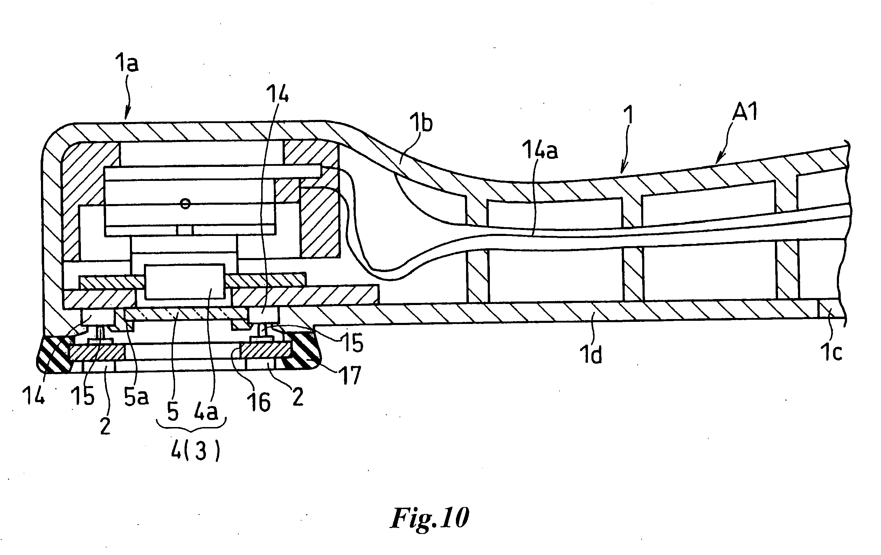

FIG. 10 shows one example of the intraoral imaging apparatus A1 in which an irradiation means is adapted to be detachable. The intraoral imaging apparatus A1 in the figure has, at the head part 1a of a main body casing 1 thereof supportable by hands and fingers, irradiation means (LED) 2 for irradiating an exciting light and an illumination light to the subject portion to be observed and the imaging means 4 as the observation section 3. The imaging means 4 is composed of the same solid state imaging device 4a described above and the light receiving filter 5 for transmitting fluorescence radiated from lesion of the subject portion to be observed and an illumination light radiated from the periphery of the lesion to thereby guide them to the solid state imaging device 4a. The irradiation means 2 is detachable from the head part 1a.

Specifically, immediately below a tip end side case 1d and the solid state imaging device 4a, a ring-like receiving section 5a for supporting the light receiving filter 5 is formed, and also near the periphery of the receiving section 5a, a plurality of receiving electrodes 14 are provided securely. Ring-like support members 16 having a plurality of projecting electrodes 15 which make electrical contact in correspondence with these receiving electrodes 14 are detachably fitted by being fitted in ring-like fitting members 17 which are fixed to the tip end side case 1d and which have an elastic rubber inner diameter. To the lower side of these ring-like support members 16, a plurality of LEDs (irradiation means) 2 are mounted which are in conductive connection with each projecting electrode 15. The LED constituting the irradiation means 2 is composed of at least: as described above, an exciting light emitting LED (exciting light emission section) for radiating fluorescence, specific to lesion, from the lesion upon irradiation of the lesion and an illumination light emitting LED (illumination light emission section). Numeral 14a denotes a lead wire for the irradiation means 2 or the solid state imaging device 4a.

Therefore, fitting the ring-like support members 16 by fitting them in the ring-like fitting members 17 connects the projecting electrodes 15 with the corresponding receiving electrode 14 accordingly, thus bringing it into an electrically conducting state. On the other hand, removing the ring-like support members 16 from the fitting members 17 disconnects the conduction between the projecting electrode 15 and the receiving electrodes 14. Therefore, replacement of the LED 2 or the like can easily be achieved by detaching the support members 16 from the ring-like fitting members 17.

According to this intraoral imaging apparatus A1, if, with the head part 1a thereof inserted in the oral cavity and with the irradiation means (exciting light emitting and illumination light emitting LEDs) 2 lit up, an exciting light and an illumination light are simultaneously irradiated to the subject portion to be observed; from lesion as the subject portion to be observed, fluorescence specific thereto is radiated. In addition, on the portion other than the lesion, the irradiated illumination light and exciting light are reflected. The fluorescence, the reflected illumination light, and the reflected exciting light radiated from the subject portion to be observed are directed to the light receiving filter 5, through which the fluorescence and the reflected illumination light are transmitted to be guided and formed on the solid state imaging device 4a. Therefore, the solid state imaging device 4a can provide information of a sharp image of a high diagnostic value including the image of healthy normal tissue at the periphery of the lesion obtained by the reflected illumination light transmitted as described above and a fluorescence image of the lesion, those images being superposed one on another.

It is also possible that, before performing diagnosis, only the white LED for an illumination light is lit up to permit observing an entire subject portion to be observed. In this case, as described above, the operator himself or herself can perform the observation without driving the imaging means 4. Moreover, in combination with an infrared light LED or an ultraviolet LED, irradiation can be performed in accordance with a diagnostic purpose, as described above. In this case, using mechanism of the irradiation means 2 which is detachable, replacement can be performed by attaching these LEDs which are separately prepared.

### THIRD EMBODIMENT

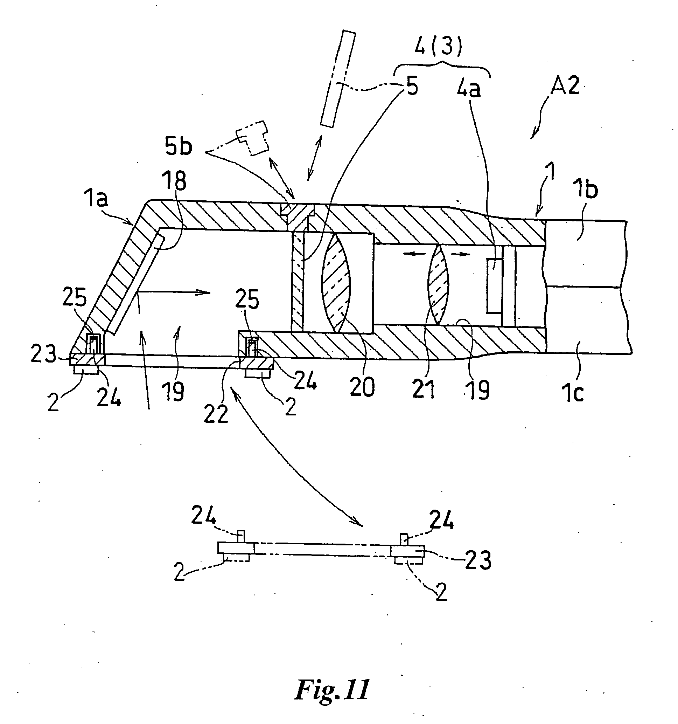

FIG. 11 shows an example of an intraoral imaging apparatus A2 having imaging means provided with optical path changing means. In the intraoral imaging apparatus A2 in the figure, a solid state imaging device 4a constituting the imaging means 4 as the observation section 3 is disposed in the head part 1a of the main body casing 1, with the optical axis of the solid state imaging device 4a extending along the longitudinal direction of the main body casing 1, and, on the tip end side inner surface of the head part 1a, a mirror (or prism) 18 as the optical path changing means is so mounted as to form an angle of substantially 45 degrees with respect to the optical axis described above, so that an inner cylindrical part between the mirror 18 and the solid state imaging device 4a is provided as a light guide path 19 for an imaged light. Disposed in this light guide path 19 are a relay lens 20; and a relay lens 21 transferable along the optical axis. The mirror 18, the relay lenses 20 and 21 described above, and the light receiving filter 5, to be described later, forming part of the observation section 3 form an optical system for forming an optical image on the solid state imaging device 4a, and this optical system and the solid state imaging device 4a construct the imaging means 4.

In the middle of the cylindrical head part 1a near the front side (tip end side) of the relay lens 20 described above, the light receiving filter 5 is detachably fitted. Numeral 5b denotes a cap for suppressing slipping of the light receiving filter 5. Therefore, providing various light receiving filters with different wavelength characteristics permits easy filter replacement in accordance with the diagnostic purpose. Zoom mechanism is constructed by adding appropriate transfer mechanism to the relay lens 21 described above along the optical axis. The relay lenses 20 and 21 are indicated in the figure as convex lenses, although not limited thereto; thus, it is needless to say that any lens is applicable which conveys the optical image. Image conveyance means such as a light guide may be of course used.

In the head part 1a, an opening 22 for an incoming light is formed which opens in a direction substantially orthogonal to the optical axis described above, so that this opening 22 and the light guide path 19 described above communicate with each other. At the periphery of the opening 22, a ring-like support member 23 is detachably mounted which has a plurality of LEDs (irradiation means) 2 arranged along the circumferencial direction thereof in such a manner as to be isolated from one another. The plurality of LEDs constituting the irradiation means 2 include at least the exciting light emitting LED (exciting light emission section) and the white light LED for an illumination light (illumination light emission section), which are capable of simultaneous emission. On the back surface of the ring-like support member 23, male receiving electrodes 24 are formed in correspondence with the LEDs 2 described above, and are so adapted as to be electrically joined with female supply electrodes 25 formed at the head part 1a when the ring-like support member 23 is fitted in the head part 1a. The engagement and joining relationship between the male and female electrodes 24 and 25 described above constructs detaching mechanism of the ring-like support member 23, which permits mounting the irradiation means 2 through one-touch operation.

The ring-like support member 23 described above can be prepared in plural types for each irradiation means 2, for example, a combination of the exciting light emitting LED (exciting light emission section) and the white light LED (illumination light emission section), only the infrared light LED or the ultraviolet light LED; and further an appropriate combination of these just mentioned, and the like. Then, the plural light receiving filters 5 are also prepared which have transmission characteristics in accordance with the irradiation means 2 used, and then are selectively fitted as appropriate. That is, when a combination of the exciting light emitting LED and the white light LED for an illumination light is used as the irradiation means 2, the one which has, as described above, such a characteristic as to cut off an exciting light but transmit fluorescence, specific to lesion, from lesion and an illumination light reflected from the healthy normal tissue of the periphery of the lesion is used as the light receiving filter 5. The irradiation of the exciting light and the illumination light by the irradiation means 2 and transmission characteristic of the light receiving filter 5 provide, as described above, an image information of a high diagnostic value in which a fluorescence image of the lesion and the image of healthy normal tissue at the periphery of the lesion appear, as described above. When the ultraviolet light is used as the illumination light, the light receiving filter is removed during use.

When a visible light image is formed on the solid state imaging device 4a with the light receiving filter 5 left fitted, a resulting image lacks some color component due to the presence of the light receiving filter 5, but an applicable image can be obtained in many cases, depending on a purpose. Further, when the infrared light LED or the ultraviolet light LED is used, the light receiving filter 5 which transmits only the infrared light or the ultraviolet light is used. It is of course possible that a plural types of LEDs are provided to the ring-like support member 23 so that any one of them is selectively irradiated, and the light receiving filter 5 described above having a wavelength characteristic in accordance with the selected LED is selectively fitted. When the light receiving filter which transmits only the infrared light or only the ultraviolet light is used, it is difficult to obtain a satisfactory image unless the filter is removed upon illumination of the white LED.

Thus, light from the irradiation means 2 is irradiated to the subject portion to be observed such as tooth, and in accordance with a wavelength characteristic of the subject portion to be observed based on the type of the irradiation means 2, the reflection light, fluorescence, or the like is radiated from the subject portion to be observed. An optical image light based on this radiation enters through the opening 22 into the head part 1a, and is reflected by the mirror 18 through 90 degrees, transmitted through the light receiving filter 5, condensed on the relay lenses 21 and 22 while traveling through the light guide path 19, and then is formed on the solid state imaging device 4a. The mounting position of the light receiving filter 5 is not limited to the position illustrated; thus, arbitrary position is possible as long as it is located on the light guide path 19. Moreover, it is needless to say that separately providing means for coupling electrical wires for the irradiation means 2 permits adopting different joining means instead of the male and female electrodes 24 and 25. Other construction is the same as described above, and thus common portions are provided with the same numerals and omitted from the description.

### FOURTH EMBODIMENT

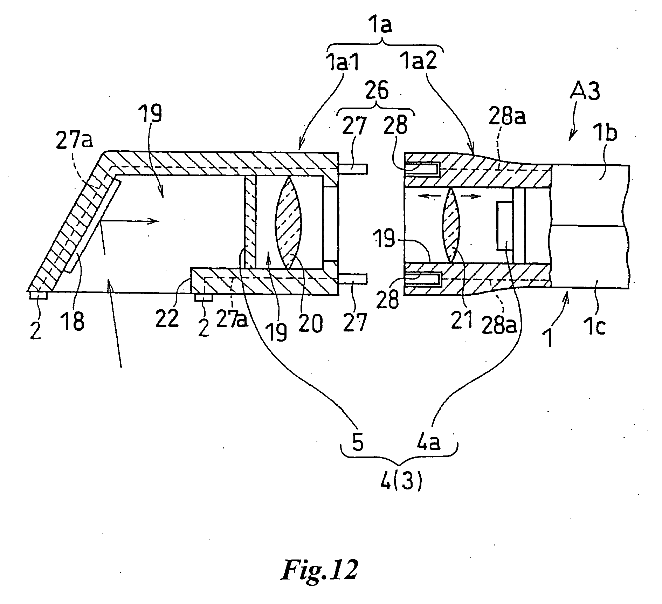

FIG. 12 shows an example of an intraoral imaging apparatus A3 having, as with the third embodiment, imaging means provided with optical path changing means and having the head part 1a separable into a head part 1a1 and a base part 1a2, with portions in common with the third embodiment provided with the same numerals. Specifically, on the inner surface of the cylindrical head part 1a1, the mirror (or prism) 18 as the optical path changing means is so mounted as to form an angle of substantially 45 degrees with respect to the optical axis described above, while, in the cylindrical base part 1a2 of the head part 1a, a solid state imaging device 4a constituting the imaging means 4 as the observation section 3 is installed. When the head part 1a1 and the base part 1a2 are linked together via link means 26 to be described later, an inner cylindrical part between this mirror 18 and the solid state imaging device 4a is provided as the light guide path 19 for the imaged light.

Along this light guide path 19, the relay lens 20 and the relay lens 21 transferable along the optical axis are disposed in the head part 1a1 and the base part 1a2, respectively. The mirror 18, the relay lenses 20 and 21 described above, and the light receiving filter 5 to be described later form an optical system for forming an optical image on the solid state imaging device 4a, and this optical system and the solid state imaging device 4a construct the imaging means 4. The opening 22 for an incoming light is formed which opens in a direction substantially orthogonal to the optical axis of the head part 1a1 described above, so that this opening 22 and the light guide path 19 described above communicate with each other. Near the front side (tip end side) of the relay lens 20, the light receiving filter 5 is fitted.

At the periphery of the opening 22, a plurality of LEDs (irradiation means) 2 are arranged along the circumferencial direction so as to be isolated from one another. The plurality of LEDs constituting the irradiation means 2 include at least the exciting light emitting LED (exciting light emission section) and the white light LED for the illumination light (illumination light emission section), which are capable of simultaneous emission. To the respective LEDs 2, lead wires 27a ... are connected which are buried in the wall of the head part 1a1, and these lead wires 27a ... are further linked to male electrodes 27 ... which protrude from the base part 1a2 side end surface of the head part 1a1. On the other hand, at the head part 1a1 side end surface of the base part 1a2, female electrodes 28 ... are provided in correspondence with the male electrodes 27 ... in a recessed manner, and these female electrodes 28 are connected to a power supply section, not shown, in the main body casing 1 via lead wires 28a... buried in the wall of the base part 1a2.

The engagement relationship between the male electrodes 27... and the female electrodes 28 ... described above constructs the link means 26 between the head part 1a1 and the base part 1a2 and also forms an electric joining section of the both electrodes, thus permitting easy detachment from the base part 1a2 through manual operation of the head part 1a1. As a result, the male electrodes 27 ...and the female electrodes 28 ... are electrically joined together, so that, through appropriate operation of the light source selection switch 7, a power is supplied from the power source section described above to the LEDs 2 whereby they are activated to emit lights.

In construction as described above, preparing various types of head parts 1a1 which combine together various types of the light receiving filters 5 having different wavelength characteristics and various types of LEDs 2 having different emission characteristics permits performing multiple diagnostic photographing in accordance with condition of the subject portion to be observed or diagnostic purposes by selectively fitting, as appropriate, these head parts 1a1 to the base part 1a2s via the link means 26. Such a manner of use is the same as that in the third embodiment, and thus the operation thereof and the like are omitted from the description. Moreover, other construction thereof is also the same as that in the third embodiment; thus, portions in common therewith are provided with the same numerals, and thus omitted from the description.

### FIFTH EMBODIMENT

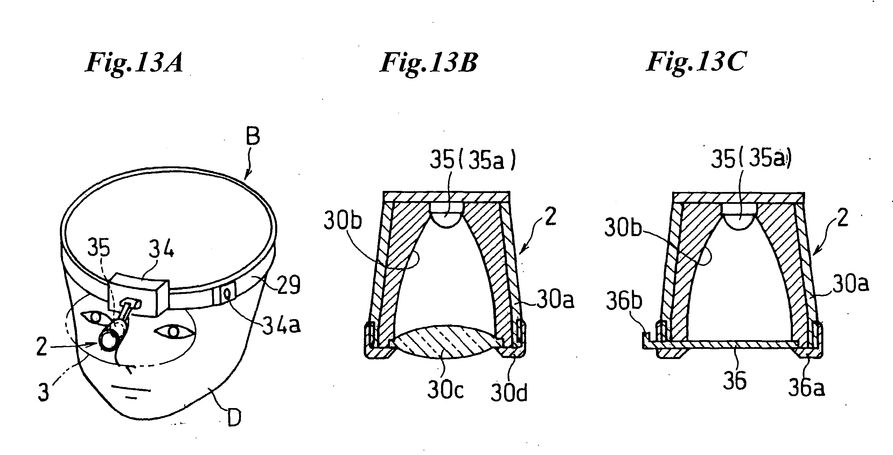

FIGS. 13A to 13C are diagram showing examples of a living body observing apparatus of the present invention embodied in a medical irradiation head device, with 13A as a conceptual diagram with the device in use, 13B as an enlarged sectional view of irradiation means, and 13C as the same diagram of a modified example. The medical irradiation head device B in the figure is composed of: head fitting means 29 fitted on the head of an operator D, and irradiation means 2 mounted on this head fitting means 29. The head fitting means 29 in the illustrated example is shown as a fastening belt or an elastic belt, although not limited thereto, and thus head fitting means of this type heretofore known in the medical field may be applicable.

The irradiation means 2 is oscillatablly fitted to a mounting base 34 provided at a position in the head fitting means 29 corresponding to the forehead portion of the operator D, and its irradiation angle with respect to an irradiation field is adjustable. A light switch 34a for turning on and off the irradiation means 2 is provided on the side part of the head fitting means 29 so as to permit easy operation by the operator D. Note that the operator D here refers to a concept including a doctor and an assistant or nurse who assists him or her in diagnostic operation. As a power source of the irradiation means 2, a battery (not shown) is used which is replaceably mounted on an appropriate area of the head fitting means 29.

The irradiation means 2 in the illustrated example is constructed with an emission section and an optical member which are packaged together. Specifically, the irradiation means 2 in FIG. 13B is composed of: an emission section 35 disposed at the bottom center of a cup-like casing 30a; a mirror 30b formed in the shape of a concave cone on the inner side wall of the casing 30a; and a condensing lens 30c mounted at the tip side opening of the casing 30a. Numeral 30d denotes a cap nut for fixing the condensing lens 30c. Lights emitted from the emission section 35, including light reflected on the mirror 30b, are transmitted through the condensing lens 30c and then irradiated to the subject portion to be observed. In this manner, the lights emitted from the emission section 35 are irradiated by the mirror 30b and the condensing lens 30c to the subject portion to be observed without any waste.

The emission section 35 in the illustrated example is shown as a bare-chip type LED 35a for dental diagnosis, which is provided with: an exciting light bare chip as an exciting light emission section for a wavelength of 400 ± 30 nm, 400 ± 30 nm or 470 ±30 nm; and a white light bare chip as an illumination light emission section. Moreover, in addition thereto, a bare chip for emitting the infrared light or the ultraviolet light may be provided.

In the illustrated example, an airspace portion (two-dot chain line portion) below the front portion of the head fitting means 29 at a position near the front of the orbit of the operator D is provided as the observation section 3. Therefore, upon diagnosis, the operator D fits the head fitting means 29 on his or her own head, turns on the switch 34a, and makes adjustment so that the irradiation field of the irradiation light from the emission section 35 is directed to a subject portion to be diagnosed. Through this switch operation, the exciting light bare chip and the white light bare chip described above simultaneously emit lights, whereby the exciting light and the illumination light are simultaneously irradiated to the subject portion to be diagnosed. Then, the condition of the subject portion to be diagnosed which has been simultaneously irradiated with the exciting light and the illumination light is observed through the observation section 3. At this point, if there is lesion in the subject portion to be diagnosed, the lesion is excited by the exciting light, thus causing fluorescence radiation specific thereto from this lesion. Moreover, a reflection light obtained by the illumination light is radiated from the periphery of the lesion. The operator D, through observation of this fluorescence and the reflection light obtained by the illumination light, can adequately recognize the condition, such as the position, degree, quality, and the like of the lesion in an outline image of the periphery of the lesion. The switch 34a is composed of one switch section so as to permit simultaneous irradiation of the two types of bare chips described above, but the bare chips may be provided individually so that they are simultaneously or individually turned on and off through operation by the operator D. Thus, construction of the observation section 3 without any glasses or goggles is also included.

FIG. 13C shows the modified example of the above embodiment, with a translucent member 36, instead of the lens 30c described above, detachably mounted at the tip side opening of the casing 30a. Numeral 36a is a cap nut for holding this translucent member 36 in a manner such that the translucent member 36 is removable and insertable from side to side. Numeral 36b is a grip for removing and inserting the translucent member 36. The translucent member 36 has no lens function as described above, but protects the bare-chip type LED 35a.

The bare-chip type LED 35a described above is provided with: an exciting light bare chip, and red, blue, and yellow (three primary light colors) bare chips. When these are simultaneously lit up, a white light synthesized with red, blue, and yellow becomes an illumination light, and, as described above, a fluorescence image of lesion obtained by an exciting light and a reflection light image from the healthy normal tissue at the periphery of the lesion obtained by the white light as the illumination light are simultaneously, clearly, visually recognized. Then, if the red, blue, and yellow ones are individually lit up and are provided as illumination lights, the image of healthy normal tissue at the periphery of the lesion can be obtained with each of the colors stressed, thus providing various diagnostic image information.

Moreover, by separately preparing a filter transmitting only a red light or a filter transmitting only a blue light and selectively fitting them instead of the translucent member 36, these lights can be selectively extracted from the white bare chip for irradiation. Moreover, an optimum exciting light corresponding to an individual case of an affected part can be selectively irradiated alone. Therefore, independently from observation and diagnosis through the simultaneous irradiation of the exciting light and the illumination light described above, these filters can be fitted as appropriate to observe the subject portion to be observed, thus achieving various diagnosis and treatments. An irradiation filter and detaching mechanism thereof in this case also has a function of selection means for selecting the type of light irradiated from the irradiation means 35.

### SIXTH EMBODIMENT

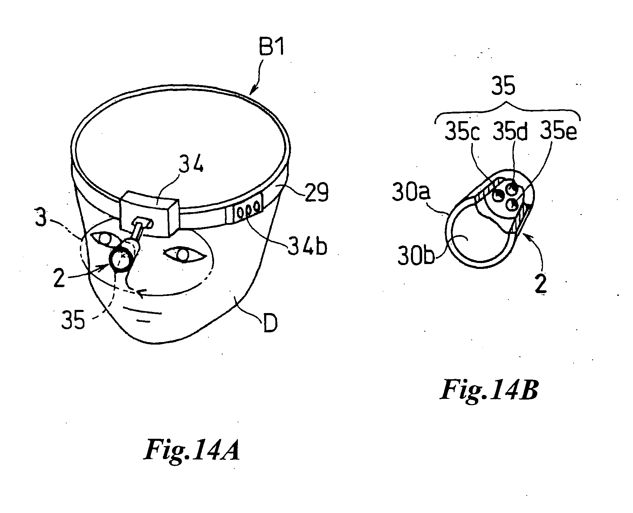

FIGS. 14A and 14B show examples of a medical irradiation head device B1 having packaged irradiation means 2 composed of a plurality of types (three types in the illustrated example) of LEDs (or bare chips) 35c, 35d, and 35e as emission sections 35. FIG. 14A is a conceptual diagram showing the device in use, and FIG. 14B is a partially cut-away enlarged perspective view showing the irradiation means 2. Specifically, the irradiation means 2 is, as described above, composed of the exciting light emitting LED (exciting light emission section) 35c, and the white light LED 35d and the red light LED35e as illumination light emission sections, which are disposed at the bottom of a cup-like casing 30a, and is oscillatably mounted on the mounting base 34. A light switch 34b is provided with three switch sections corresponding to these emission sections 35, and the operator D can selectively operate these switch sections to irradiate a desired light in accordance with a diagnostic purpose. Operation of turning on the switch section for the exciting light emitting LED 35c and the switch section for the white light LED 35d, in particular, can be performed simultaneously.

On the inner wall of the casing 30a, the same mirror 30b as described above is provided. In addition, although omitted from the figure, the condensing lens described above is desirably adopted to be fitted to the tip side opening. To selectively cause the LEDs 35c, 35d, and 35e described above constituting the emission sections 35 to emit light, it is needless to say that an adequate light receiving filter may be selectively fitted thereto for use.

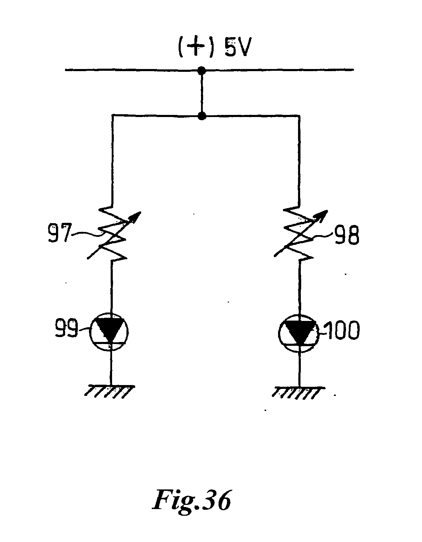

The number of emission sections 35 is not limited to three, and thus may be two, or four or more. In addition, for the type of emission sections 35, an LED emitting light of a particular wavelength other than those described above can be adopted. Moreover, as described later in the description of FIG. 36, by making light amount adjustment with a variable resistor, the ratio of light amount between the exciting light and the illumination light can be changed to adjust the affected part to be more easily visually recognized together with the healthy normal tissue. Other construction and operation are the same as those described above, and thus portions common therewith are provided with the same numerals and omitted from the description.

### SEVENTH EMBODIMENT

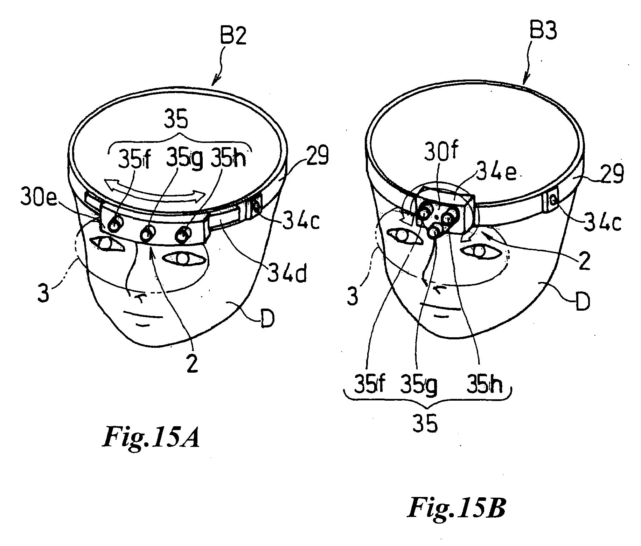

FIGS. 15A and 15B show examples of medical irradiation head devices B2 and B3 adapted to change the position of a plurality of emission sections 35 to thereby selectively perform irradiation. The medical irradiation head device B2 in FIG. 15A has three bare-chip type LEDs 35f, 35g, and 35h as the emission sections 35 disposed serially at equal intervals on a horizontally long slide member (mounting base) 30e, which is slidably mounted along the longitudinal direction of a receiving guide member 34d extending along the longitudinal direction of head fitting means 29. On the longitudinal central part of the receiving guide member 34d, one pair of electrical contacts (not shown) is provided. On the back surface of the slide member 30e, contacts (not shown) respectively corresponding to the LEDs 35f, 35g, and 35h are provided. Sliding the slide member 30e causes any one of the contacts respectively corresponding to the LEDs 35f, 35g, and 35h, which is consequently located at the center, to join electrically with the electrical contacts on the receiving guide member 34d side. Therefore, operation of turning on a light switch 34c causes the emission section 35 establishing this electrical joining to light up.

Each of the LEDs 35f, 35g, and 35h, as with the fifth embodiment, is provided with an exciting light emitting bare chip (exciting light emission section) and a white light bare chip for illumination (illumination light emission section). The exciting light emitting bare chips of the LEDs 35f, 35g, and 35h have mutually different emission wavelengths. These three types of emission wavelengths are, for dental treatment for example, 400± 30 nm, 400 ± 30 nm or 470 ± 30 nm described above. Therefore, the operator D, upon diagnosis, slides the slide member 30e from side to side through manual operation, locates a desired emission section 35 at the center in accordance with a characteristic of lesion, and turns on the light switch 34c, whereby the exciting light bare chip and the white light bare chip for illumination of the located emission section 35 light up simultaneously. Specifically, an exciting light and a white light for illumination of particular wavelengths from the selected emission section 35 are simultaneously irradiated to the subject portion to be observed, and fluorescence specific to lesion based on the irradiated exciting light and a reflection light image based on the irradiation of the white light for illumination are simultaneously, clearly, visually recognized. Then, in accordance with the characteristic of the lesion, the slide member 30e is manually operated and the LEDs 35f, 35g, and 35h are selectively lit up as appropriate, thereby performing various observation and diagnosis.

The medical irradiation head device B3 of FIG. 15B has a mounting base 34e securely provided on the forehead side central part of head fitting means 29. On a rotary member (mounting base part) 30f rotatably mounted on this mounting base 34e, the same three bare-chip type LEDs 35f, 35g, and 35h as described above as the emission sections 35 are disposed at equal intervals along the circumference concentric with the rotation center of the rotary member. At position corresponding to the rotating orbit of the emission section 35 on the mounting base 34e, one pair of electrical contacts (not shown) is provided. On the back surface of the rotary member 30f, contacts (not shown) respectively corresponding to the LEDs 35f, 35g, and 35h are provided. Rotating the rotary member 30f causes any one of the contacts corresponding to the LEDs 35f, 35g, and 35h to electrically join with the electrical contacts on the mounting base 34e side. Therefore, operation of turing on the light switch 34c causes the emission section 35 establishing this electrical joining to light up.

Therefore, the operator D, upon diagnosis, rotates the rotary member 30f through manual operation, locates a desired emission section 35 at the joining position described above, and turns on the light switch 34c, whereby the exciting light bare chip and the white light bare chip for illumination of the located emission section 35 simultaneously are lit up. The sliding from side to side by the slide member 30e described above and rotation by this rotary member 30f correspond to means for selecting irradiation means, and they can be adapted to be driven by the motor.

### EIGHTH EMBODIMENT

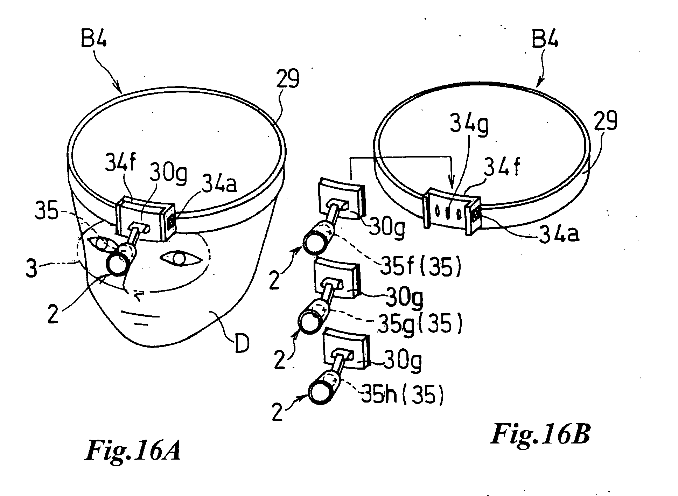

FIGS. 16A and 16B show examples of a medical irradiation head device B4 having irradiation means 2 detachably mounted on to head fitting means 29, with FIG. 16A as a conceptual diagram with the device in use and FIG. 16B as a diagram showing detaching mechanism. On the forehead side central part of the head fitting means 29, a mounting base 34f of a concave shape in cross section is securely provided with a pair of electrical contacts 34g provided at the bottom thereof. The irradiation means 2 is constructed as a packaged type having therein the same emission sections 35 as described above, and is oscillatably mounted on a mounting base part 30g. This mounting base part 30g is so shaped as to be held by being fitted in the concave part of the mounting base 34f. On the back surface of the mounting base part 30g, contacts (not shown) are provided which are paired with the electrical contacts 34g described above. When the irradiation means 2 is fitted via the mounting base part 30g to the mounting base 34f, the both contacts are electrically joined together. On the side part of the mounting base 34f, a light switch 34a is provided, which is operated to light up the emission section 35 of the fitted irradiation means 2.

FIG. 16B shows three types of irradiation means 2 are prepared which can be selectively and appropriately fitted to the head fitting means 29. In the illustrated example, the three types of irradiation means 2 are provided with the bare-chip type LEDs 35f, 35g, and 35h as the emission sections 35. Each of these LEDs 35f, 35g, and 35h, as described above, is provided with: an exciting light emitting bare chip (exciting light emission section) and a white light bare chip for illumination (illumination light emission section). The exciting light emitting bare chips of the LEDs 35f, 35g, and 35h have mutually different emission wavelengths. Therefore, the operator D, in accordance with the purpose of observation and diagnosis, can select a desired irradiation means 2, fit the mounting base part 30g by engaging it in the mounting base 34f from above as shown in the figure, turn on the light switch 34a, and simultaneously irradiate the same exciting light and illumination light as described above to thereby achieve observation and diagnosis of the subject portion to be observed. Moreover, various observations and diagnosis as described above can be performed by selectively and appropriately replacing these three types of irradiation means 2. Needless to say, as the emission section 35, the one having a different wavelength characteristic can be replaced or added.

### NINTH EMBODIMENT

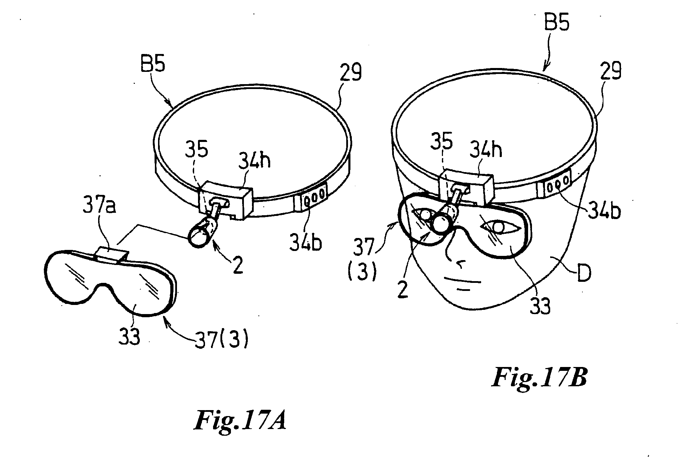

FIGS. 17A and 17B show examples of a medical irradiation head device B5 having the observation section 3 formed of a translucent glass lens section such as protective glasses, or a goggle main body section such as protective goggles. As a medical appliance, special glasses or goggles are used for protecting the operator's eyes from droplets of a treatment solution or preventing infection. The medical irradiation head device B5 of the present embodiment is even provided with such a function of protecting the eyes. Here, the glass lens section and the goggle main body section described above are collectively termed as an orbit protective member 37. FIG. 17A shows mechanism of detaching the orbit protective member 37, and FIG. 17B is a conceptual diagram of the member in use.

On the forehead side central part of the head fitting means 29, a mounting base 34h is securely provided. On the mounting base 34h, irradiation means 2 is oscillatably mounted which packages the same emission sections 35 emitting three types of lights as the sixth embodiment. Provided on the side part of the head fitting means 29 is a light switch 34b provided with three switch sections which permit selectively causing the three types of emission sections 35 to light up. The orbit protective member 37 is detachably fitted via a mounting base part 37a thereof to the lower side part of the mounting base 34h as shown by an arrow. The orbit protective member 37 may be just formed of a translucent member, but it may be of the type which permits providing an appropriate degree in accordance with the visual acuity of the operator. The light transmission portion of the orbit protective member 37 is provided as the light receiving filter 33 having the same transmission characteristic as described above.

Specifically, the orbit protective member 37 is formed of a member which, when the exciting light emitting LED 35c of the emission section 35 (see FIG. 14) and the white LED 35d for an illumination light (see FIG. 14) are simultaneously caused to emit lights, transmits the fluorescence from the lesion obtained through the irradiation of the exciting light and the reflection light from the periphery of the lesion obtained through the irradiation of the illumination light but also cuts off a reflected exciting light. Therefore, if the exciting light and the illumination light are simultaneously irradiated from the irradiation means 2 and the image of the subject portion to be observed is observed through the light receiving filter 33, an fluorescence image radiated from the lesion and an outline image are sharply visually recognized, as described above.

In the example described above, the orbit protective member 37 is formed of the member which permits the orbit protective member 37 itself to function as the light receiving filter 33. Alternatively, the orbit protective member 37 may be just formed of a light transmission member, and the surface thereof may be subjected to coating to provide it with the transmission (cutoff) characteristic described above, or, on the front surface or the back surface thereof, a filter member having the same transmission characteristic may be detachably fitted. Instead of the detachable mechanism by the mounting base part 37a described above, mechanism can be adopted by which the orbit protective member 37 is raised upward to be retracted. Other construction is the same as that of the sixth embodiment, and thus common portions are provided with the same numerals and omitted from the description.

### TENTH EMBODIMENT

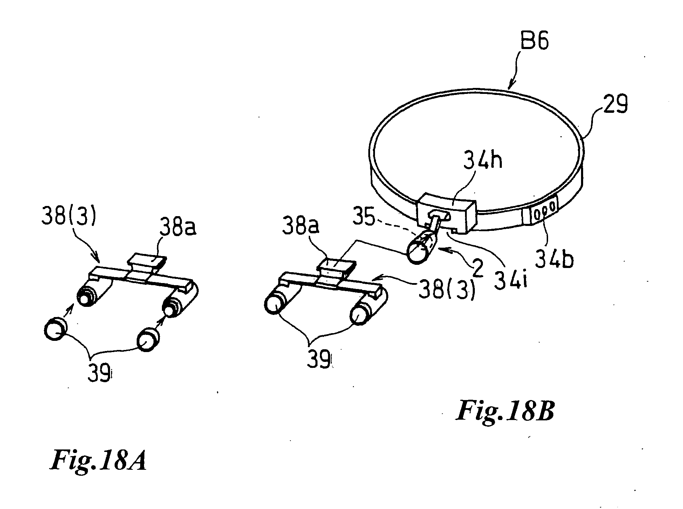

FIGS. 18A and 18B show examples of a medical irradiation head device B6 having the observation section 3 formed of a magnifying lens. The magnifying lens in the illustrated examples is exemplified by a binocular telescope 38, but may be a magnifying glass. FIG. 18A is a perspective view of the binocular telescope 38, and FIG. 18B is a diagram showing mechanism of detaching the binocular telescope 38. The irradiation means 2 packages therein emission sections 35 emitting three types of lights as with the sixth embodiment, and the irradiation means 2 is oscillatably mounted on head fitting means 29 via the mounting base 34h. Provided on the side part of the head fitting means 29 is the light switch 34b provided with three switch sections as described above.

The binocular telescope 38 is detachably fitted via a mounting base part 38a thereof to a lower side part 34i of the mounting base 34h as shown by an arrow. On the objective lens side of the binocular telescope 38, a light receiving filter 39 is detachably fitted. The light receiving filter 39 is formed of a member having a transmission (cutoff) characteristic which, as described above, when the exciting light is irradiated from the irradiation means 2 to the subject portion to be observed, transmits fluorescence radiated from the lesion and the illumination light reflected on the periphery of the lesion but also cuts off the reflected exciting light. Therefore, according to the medical irradiation head device B6 of the present embodiment, the exciting light emission section and the illumination light emission section of the irradiation means 2 can be caused to simultaneously emit lights, whereby the exciting light and the illumination light can be simultaneously irradiated to the subject portion to be observed, and then an extracted fluorescence image of the lesion and the reflection light image of the periphery of the lesion can be sharply visually recognized while being enlarged by operation of the binocular telescope 38, thus permitting accurately grasping the condition of lesion.

### ELEVENTH EMBODIMENT

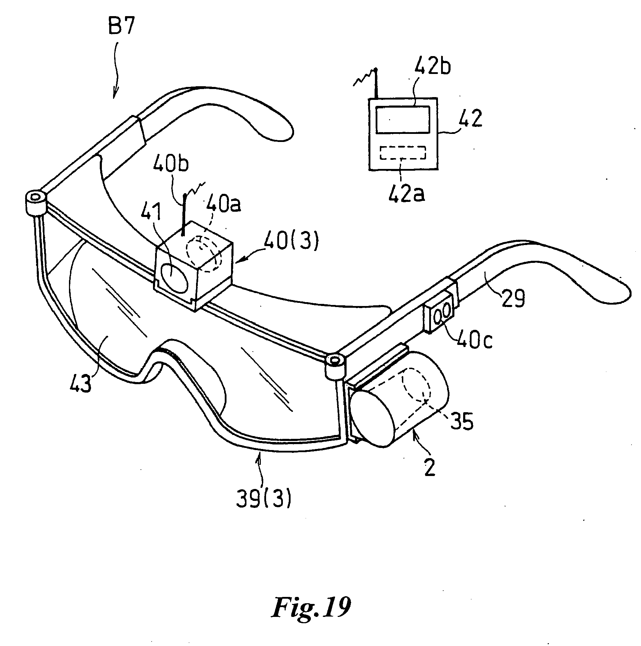

FIG. 19 shows an example of a medical irradiation head device B7 which is constructed as a whole in the form of goggles or glasses having the observation section 3 composed of the translucent goggle main body section or a glass lens section (hereinafter, referred to as the orbit protective member as described above) 39 and imaging means 40a provided with a built-in solid state imaging device 40a, such as a CCD and a MOS. The head fitting means 29 is formed of a goggle frame or a glass frame which can be fitted on the operator's ears, and this is hereinafter called a frame 29. On the side part of the frame 29, the irradiation means 2 is detachably fitted which packages therein the emission sections 35 emitting three types of lights as with the sixth embodiment. On the upper side central part of the frame 29, the imaging means 40 provided with the built-in CCD 40a is detachably set. On the light receiving section of the imaging means 40, a light receiving filter 41 is detachably fitted which has the same transmission (cutoff) characteristic as described above.

The imaging means 40 described above is further provided with a radio signal transmission section 40b, so that image data converted into an electrical signal by the CCD 40a is wirelessly transmitted to a personal computer 42 separately installed. The personal computer 42 can be configured so that a control section 42a thereof can store the received image data into a storage section or perform appropriate image processing on the image data and display it on a display 42b, and further print out the image data by a printer (not shown). On the side part of the frame 29, a switch 40c is mounted which combines both a switch for the irradiation means 2 and a drive switch for the image means 40. Note that irradiation drive control of the irradiation means 2 can also be performed by the control section 42a of the personal computer 42 described above, and this radiation drive control can be associated with image data from the imaging means 40 to thereby perform various image information processing, which is to be described later. Moreover, needless to say, the imaging means 40 and the personal computer 42 can also be connected together with wires.

The orbit protective member 39, as described above, protects the operator's eyes from droplets of a treatment solution or prevents infection, and functions as the observation section 3, whose light transmission part is provided with a member having the same transmission (cutoff) characteristic as described above and then provided as a light receiving filter 43 as in the figure. The light receiving filter 41 fitted to the imaging means 40 described above and this light receiving filter 43 have such a transmission (cutoff) characteristic that, when the exciting light and the illumination light are irradiated from the irradiation means 2 to the subject portion to be observed, transmits fluorescence radiated from the lesion and the reflection light from the periphery of the lesion but also cuts off the reflected exciting light. An image visually recognized through the orbit protective member 39 or obtained by the imaging means 40 is of a high diagnostic value including both the image of the lesion and an outline image which can be clearly recognized.

According to the medical irradiation head device B7 of the present embodiment, the operator can cause the irradiation means 2, through operation of the switch 40c, to simultaneously irradiate the exciting light and the illumination light to the subject portion to be observed, view by himself or herself the light image from the subject portion to be observed through the orbit protective member 39 and the same image canbe converted into an electrical signal by the imaging means 40 and transmitted to the personal computer 42, and then displayed on the display 42b or the like. Therefore, the patient or the assistant can, simultaneously with the operator, observe condition of the subject portion to be observed by the displayed image, which achieves communication among the operator, the patient, and the assistant, thus permitting performing highly accurate diagnosis and treatment. Image information obtained from the image means 40 is saved as carte information, which can also be effectively used for continuous diagnosis and the like. Moreover, various diagnostic image information can also be obtained by selectively and appropriately using the light receiving filter 43 of the orbit protective member 39 and the light receiving filter 41 of the imaging means 40 and by appropriately switching emission among the emission sections 35 in the irradiation means 2.

### TWELFTH EMBODIMENT

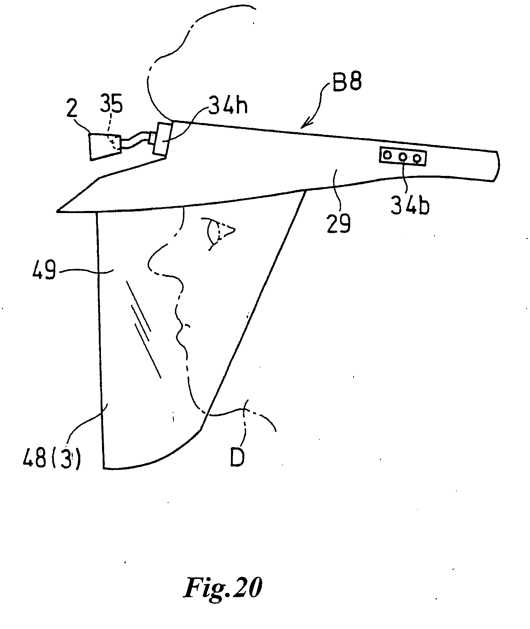

FIG. 20 shows an application example of a medical irradiation head device B8 used as a face cover for use in a laser treatment or the like. On the front surface (forehead side) of the face cover frame (visor frame) as head fitting means 29, a face cover main body 48 of translucent resin is detachably mounted which covers substantially a face of the operator D. This face cover main body 48 is provided as the observation section 3, and the surface of the face cover main body 48 is subjected to coating treatment and thus provided as a light receiving filter 49 having a transmission characteristic as described above. Needless to say, the face cover main body 48 itself can be adapted to just transmit light, and a filter member having the same transmission characteristic can be detachably fitted on the front surface or back surface of the face cover main body 48 so that this member can be provided as a light receiving filter.

On the forehead side central part of the head fitting means 29, the mounting base 34h is securely provided. On the mounting base 34h, irradiation means 2 is oscillatably and further detachably fitted which packages therein the emission sections 35 emitting three types of lights as with the sixth embodiment. Provided on the side part of the head fitting means 29 is the light switch 34b provided with three switch sections which permits selectively causing the three types of emission sections 35 described above to light up. Needless to say, on the tip side of the irradiation means 2, as described above, the irradiation filter or the condensing lens can be fitted when necessary. The light switch 34b can cause the exciting light emission section and the illumination light emission section of the emission section 35 to simultaneously emit lights. Through the simultaneous irradiation of the exciting light and the illumination light to the subject portion to be observed, as described above, the lesion and the periphery thereof can be clearly visually recognized through the light receiving filter 49.

### THIRTEENTH EMBODIMENT

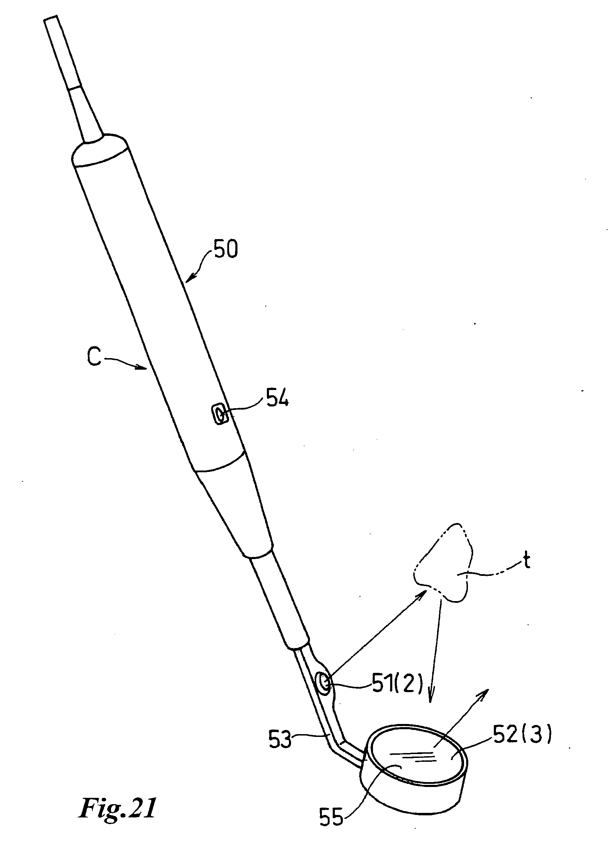

FIG. 21 shows a living body observing apparatus of the present invention embodied in a dental mirror. In the illustrated example, the dental mirror C is composed of a main body 50 supportable by hands and fingers and insertable in the oral cavity, irradiation means 2, and a mirror section 52 on the tip side. The irradiation means 2 is installed in the middle of a support arm 53 supporting the mirror section 52 on the tip side of the main body 50 at a predetermined angle, and is constructed with an emission section 51composed of bare-chip type LEDs emitting the exciting light and the illumination light having the same wavelength characteristics as described above. This is lit up through operation of a light switch 54 provided at the trunk of the main body 50 while using a battery (not shown) built in the main body 50 as a power source. Light emitted from the irradiation means 2 is adapted to be directed to the tooth t as shown by an arrow so that light radiated from this tooth t is then directed to the mirror section 52. The mirror section 52 is provided as the observation section 3, and the operator observes an image reflected by the mirror section 52 to perform diagnosis.

The mirror section 52 has, on a surface thereof, a light receiving filter 55 formed by coating the surface with such a coating agent as to transmit fluorescence generated by the subject portion to be observed (the tooth t) and part of the reflected exciting light or by attaching to the surface a translucent resin film having the same transmission (cutoff) characteristic. The dental mirror C of the present embodiment, as with a conventionally heretofore known dental mirror, is designed to have a tip side thereof inserted in the oral cavity to permit observation and diagnosis of condition of the tooth t reflected on the mirror section 52.

Thus, with the dental mirror C of the present embodiment, upon the observation and diagnosis described above, the light switch 54 is turned on, and the exciting light and the illumination light are simultaneously irradiated by the irradiation means 2 to the tooth t as the subject portion to be observed. If there is lesion such as caries, in the tooth t, fluorescence specific thereto obtained through the irradiation of the exiting light is radiated. The irradiated illumination light is reflected on the region other than the lesion. These lights are directed to the mirror section 52, on which the light receiving filter 55 having the transmission (cutoff) characteristic described above is formed, and the fluorescence is transmitted through this light receiving filter 55 and reflected on the mirror section 52. The illumination light reflected on the periphery of the lesion is also transmitted through the light receiving filter 55 and reflected on the mirror section 52. Then, most of the exciting light reflected on the periphery of the lesion is cutoff (absorbed) by the light receiving filter section 55. Therefore, an fluorescence image reflected from the mirror section 52 is a sharp image, and further an outline image of the tooth t obtained by the illumination light transmitted through the light receiving filter 55 and reflected is also visually recognized, thus permitting the relative position and degree of the lesion in the tooth t to be adequately recognized, which is extremely useful for the diagnosis and treatment.

### FOURTEENTH EMBODIMENT

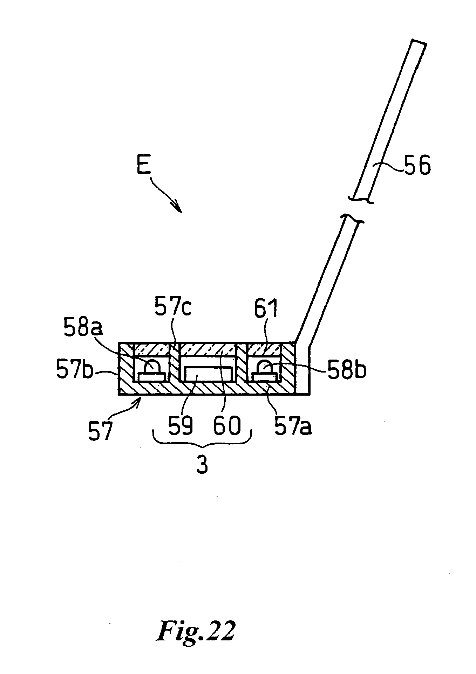

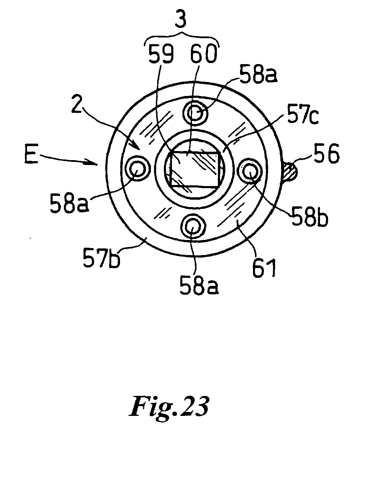

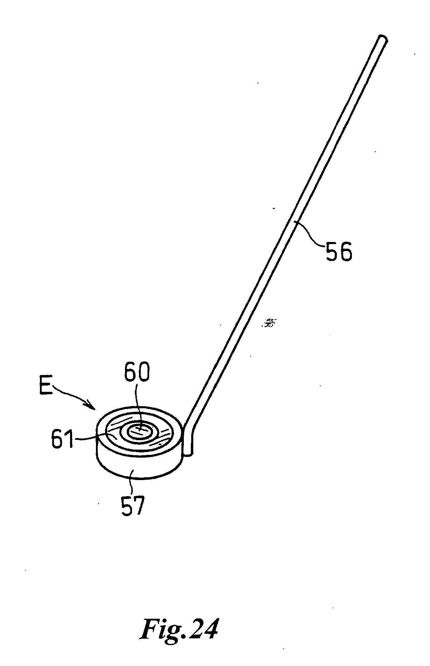

FIGS. 22 to 24 show examples of a living body observing apparatus constructed in the form of a dental mirror type. The living body observing apparatus E in the illustrated examples is constructed into a compact dental mirror type having three exciting light emitting LEDs 58a as exciting light emission sections and one white light LED 58b as an illumination light emission section which compose the irradiation means 2, a CCD 59 as imaging means, and a light receiving filter 60, arranged in a frame 57 mounted at the tip end of a support rod section 56 gripped by hands and fingers. This CCD 59 and the light receiving filter 60 compose the observation section 3. The frame 57 is composed of a circular bottom wall 57a, a cylindrical outer peripheral wall 57b, and a cylindrical partition wall 57c. Between the outer peripheral wall 57b and the partition wall 57c, four LEDs 58a and 58b are arranged, and on the inner side of the partition wall 57c, the CCD 59 is arranged.

The four LEDs 58a and 58b are arranged around the axial center of the CCD 59 at equal intervals of 90 degrees, and covered by a toric transparent glass 61 installed between the outer peripheral wall 57b and the partition wall 57c. In the inner side of the partition wall 57c, the discoid light receiving filter 60 for the CCD 59 is provided, also serving as a cover. This light receiving filter 60 has, as described above, a transmission (cutoff) characteristic which transmits the fluorescence from the lesion and the illumination light reflected on the periphery of the lesion but cuts off the reflected exciting light.

The living body observing apparatus E constructed in such a manner is used for, for example, observing condition of a tooth or condition of the oral cavity, with the frame 57 inserted in the oral cavity. When a light switch, not shown, is turned on, the three exciting light emitting LEDs 58a and the one white light LED 58b simultaneously emit lights, whereby the exciting lights and the illumination light are simultaneously irradiated to a subject portion to be observed. If there is the lesion in the subject portion to be observed, fluorescence specific thereto obtained through the irradiation of the exciting light is radiated, and the illumination light is reflected on the periphery of the lesion, and then these radiation lights are formed on the CCD 59 as imaging means, converted into electrical signals, and transmitted to a personal computer or the like, where the image are reproduced.

Since the light receiving filter 60 described above is installed at the light receiving section of the CCD 59, the fluorescence from the lesion and the illumination light reflected on the periphery thereof are transmitted and enter the CCD 59, while the exciting light reflected on the periphery of the lesion does not enter the CCD 59. Therefore, on the CCD 59, a sharp fluorescence image not masked by the reflected exciting light and an outline image of the periphery of the lesion obtained by the reflected illumination light are formed, thus providing a diagnostic image of a high practical value. As described above, the living body observing apparatus E is very compact; thus, a subject portion to be diagnosed (subject to be photographed), such as the oral cavity, can be photographed easily and conveniently with favorable operability through the same operation as performed to use the dental mirror. Part of the transparent glass 61 may be provided as an illumination filter.

### FIFTEENTH EMBODIMENT

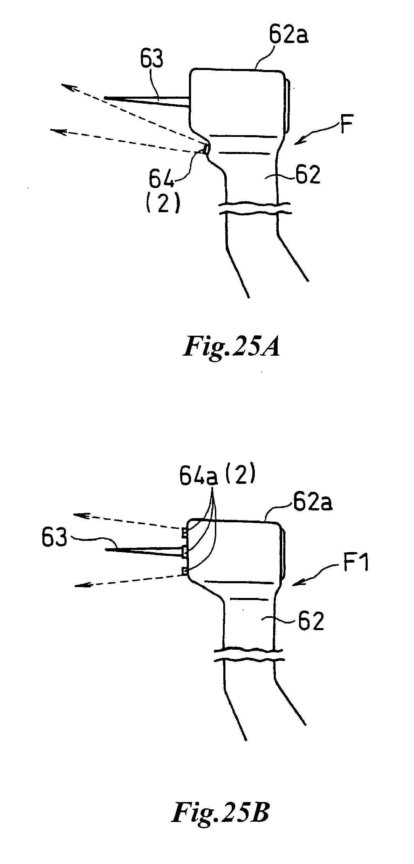

FIGS. 25A, 25B, 26A, 26B, 26C, 27, 28A, 28B, 29A, and 29B show examples of a medical treatment appliance into which the living body observing apparatus described above is integrally incorporated. The medical treatment appliance in the illustrated example is illustrated as an example of its application to a tooth cutting handpiece (air turbine handpiece or micromotor handpiece). FIG. 25A shows one of the examples, and FIG. 25B shows a modified example thereof In the dental handpiece F of FIG. 25A, to a head part 62a of a handpiece main body 62, a tooth cutting tool 63 is fitted in such a manner as to be axially rotatable and detachable by an air turbine or a micromotor. Into the neck part of this head part 62a, the irradiation means 2 provided with the exciting light emission section and the illumination light emission section is incorporated, with an irradiation field thereof directed, as shown by arrows, forward of the tip end of the cutting tool 63 described above. The irradiation means 2, in the illustrated example, is composed of, as described above, a bare-chip type LED 64 provided with the exciting light emitting bare chips (exciting light emission section) and the white light emitting bare chip (illumination light emission section).

The dental handpiece F of this type is used in cutting and removing treatment of the lesion of the tooth which is performed under the condition that the head part 62a is inserted in the oral cavity and then the cutting tool 63 in high-speed rotation is caused to act on the lesion. Thus, in the dental handpiece F with this construction, before the cutting treatment or during the cutting treatment, the exciting light emission section and the illumination light emission section of the irradiation means 2 simultaneously emit lights to simultaneously irradiate the lesion and the periphery thereof with the exciting light and the illumination light. Then, fluorescence is radiated from the lesion through the irradiation of the exciting light and the illumination light is reflected on the periphery of the lesion through the irradiation of the illumination light, and the operator performs the treatment operation while observing these radiation light images. Thus, the viewing field of the subject to be treated is bright and the outline image can be clearly visually recognized, and further the position and degree of the lesion can also be adequately grasped, thus dramatically improving the accuracy in the treatment.

The dental handpiece F1 shown in FIG. 25B has a plurality of LEDs 64a ... which are disposed around the position where the cutting tool 63 is fitted in the head part 62a and which compose the irradiation means 2. The plurality of LEDs 64a ... are composed of at least an exciting light emitting LED (exciting light emission section) and a white light LED for illumination light emission (illumination light emission section). Further, when necessary, the infrared light LED, the ultraviolet light LED, or the like can also be included, thus permitting more multiple observations and diagnosis through image information based on the characteristics of irradiation lights from these LEDs.

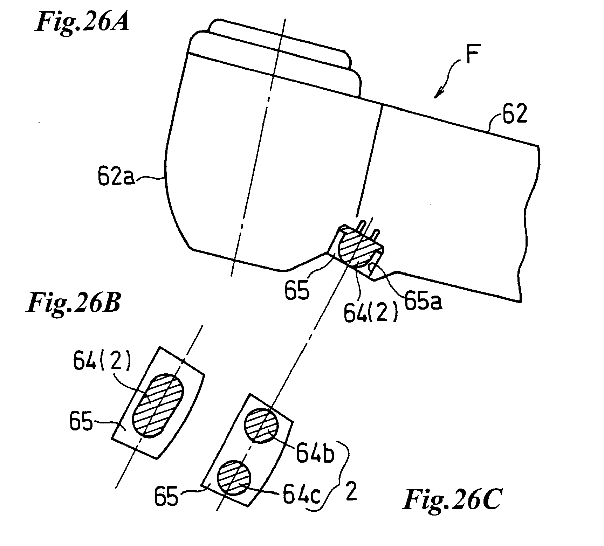

FIGS. 26A and 26B show, on an enlarged scale, the essential parts in the example of FIG. 25A, and FIGS. 26C and 27 show modified examples thereof. At the neck part of the head part 62a of the handpiece main body 62 in the dental handpiece F of FIG. 26A, a depressed portion 65 is formed which is so opened as to face the tip side of the cutting tool 63, and into this depressed portion 65, the bare-chip type LED 64 described above is detachably fitted, thereby constituting the irradiation means 2 provided with the exciting light emission section and the illumination light emission section. On the inner wall surface of the depressed portion 65, a reflective material 65a such as plating is so formed as to be deposited thereon to reduce loss of an irradiation light. To the opening of the depressed portion 65, a transparent protective member may be fitted.

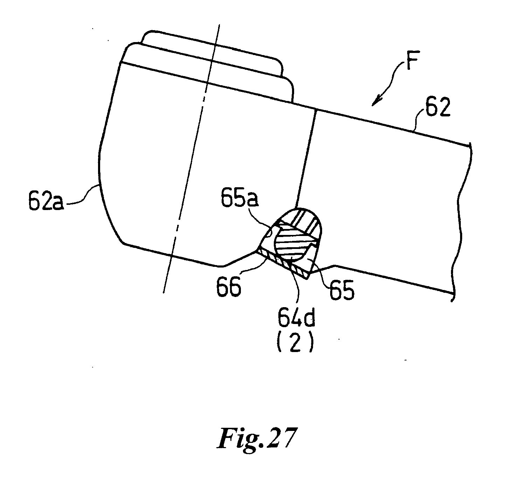

FIG. 26C shows the example of irradiation means 2 composed of an exciting light emitting LED (exciting light emission section) 64b and a white light LED (illumination light emission section) 64c. The example of FIG. 27 shows the exciting light emission section, constituting the irradiation means 2, is formed of a combination of a lamp 64d, such as a halogen lamp, covering a broad wavelength range, and an irradiation filter 66. The irradiation filter 66 extracts as an exciting light, from among lights in broad wavelengths ranges emitted from the lamp 64d, the light of the wavelength described above. Note that, on the rear or front side on the paper surface, a lamp or a LED for the illumination light emission section is provided, although omitted from the illustration for convenience. Through operation of the light source selection switch 7 described above, selection of an irradiation light source is made from among: the illumination light only, the exciting light only, and the exciting light and the illumination light to be simultaneously irradiated.

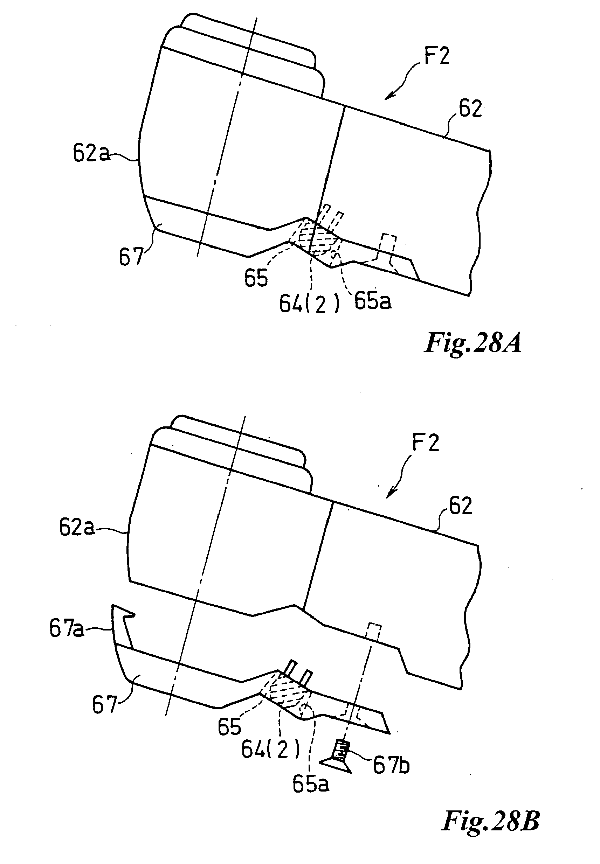

In the example shown in FIGS. 26A to 26C or 27, the emission section itself, such as the LED or the lamp, is detachable. Therefore, these emission sections are provided with an inserting terminal as in the figures, and they are fitted by inserting the inserting terminal in a female terminal provided on the bottom of the depressed portion 65. FIGS. 28A and 28B show the modified examples of such detaching mechanism of a dental handpiece F2, in which irradiation means 2 is adapted to be detachably fitted to the handpiece main body 62 via a mounting bracket 67. Specifically, the mounting bracket 67 is designed to be mounted on the cutting tool fitting side of the head part 62a, and fitted to the handpiece main body 62 by a latching pawl 67a to be retained on the outer wall part of the head part 62a and by a screw 67b to be firmly screwed into the handpiece main body 62. At the mounting bracket 67, the same depressed portion 65 and reflective material 65a as described above are formed, and to this depressed portion 65, the same emission section 64 as described above is detachably fitted, thereby constituting the irradiation means 2. When the mounting bracket 67 is fitted to the handpiece main body 62, the terminal of this emission section 64 electrically joins with a female terminal formed at the handpiece main body 62.

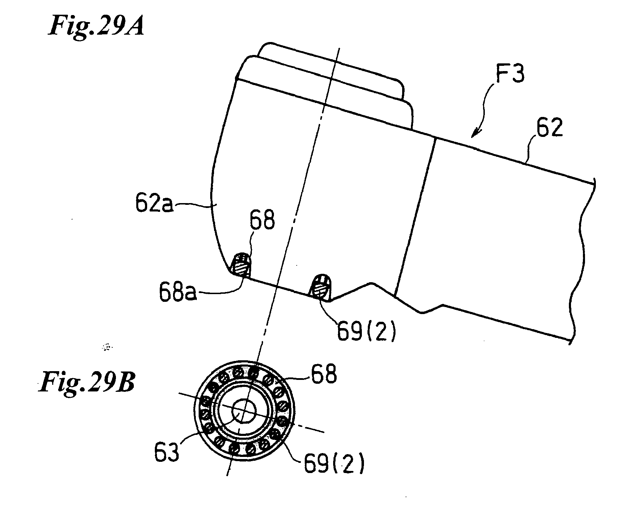

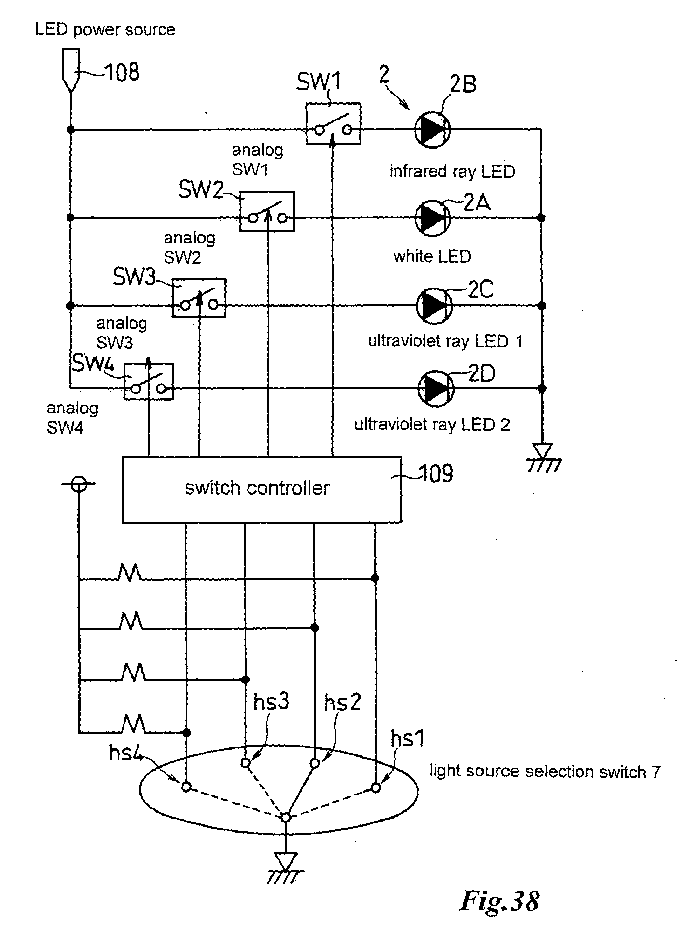

FIGS. 29A and 29B show a dental handpiece F3 as the modified example of FIG. 25B, in which irradiation means 2 is constructed with circumferential grooves 68 formed on the cutting tool fitting side end surface of the head part 62a concentrically with the cutting tool 63 and with an emission section 69 composed of a large number of LEDs arrayed in the circumferential grooves 68. The emission section 69 includes at least the exciting light emitting LED (exciting light emission section) and the white LED for illumination light emission (illumination light emission section), which are capable of simultaneous emission. Further, the infrared light LED or the ultraviolet light LED can also be added so that these LEDs are selectively caused to emit light through operation of a light source switch or an analog switch to be described later in FIG. 38. On the inner wall of the circumferential groove 68, a reflective material 68a as described above is so formed as to be deposited thereon. Instead of the circumferential grooves 68, depressed portions can be formed which permit respective LEDs to be stored therein individually.

### SIXTEENTH EMBODIMENT

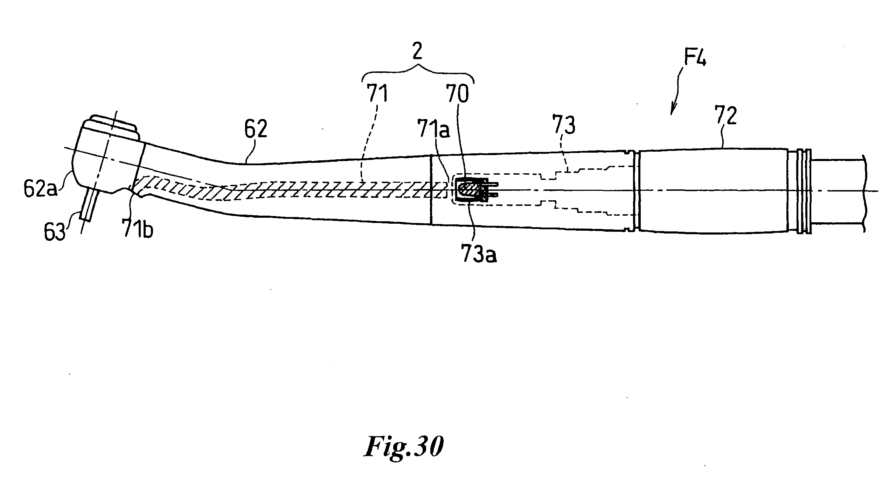

A handpiece F4 shown in FIG. 30 has the irradiation means 2 composed of: a bare-chip type LED as an emission section 70, same as that described above, disposed in the trunk of the handpiece main body 62; and a light guide body 71 formed of a glass fiber or the like. The light guide body 71 has: an entrance surface 71a which faces the light exiting section of the emission section 70: and an exit surface 71b which is arranged at the neck part of the head part 62a and through which a light guided by the light guide body 71 is irradiated forward of a cutting tool 63 from the exit surface 71b. The bare-chip type LED constituting the emission section 70 is provided with at least the same exciting light emitting bare chip (exciting light emission section) and the white light emitting bare chip (illumination light emission section), which are capable of simultaneous emission.

Numeral 72 denotes a connecter section on the hose side provided with a pipe line (not shown) for feeding an action medium, such as an electric power, air, or cooling water, for activating the cutting tool 63, or a drug solution. In a depressed portion 73a formed at the tip end of a joint section 73, the bare-chip type LED 70 described above is detachably stored and held. When the hose side connector section 72 is joined with the handpiece main body 62 via this joint section 73, the light exiting section of the bare-chip type LED 70 described above faces the entrance surface 71a of the light guide body 71, and also the hose side pipe line for feeding an action medium or a drug solution described above and a pipe line (not shown) for feeding an action medium or a drug solution formed in the handpiece main body 62 communicate with each other.

The dental handpiece F4 in this example differs from the dental handpieces F to F3 in the embodiments described above in that light from the bare-chip type LED 70 is irradiated to a treated portion through the light guide body 71, but does not differ therefrom in the operation of simultaneous irradiation of the exciting light and the illumination light by the irradiation means 2, and the like. In this case, droplets of a treatment solution or the like during tooth cutting operation does not directly reach the bare-chip type LED 70, which is advantageous in achieving longer life thereof.

### SEVENTEENTH EMBODIMENT

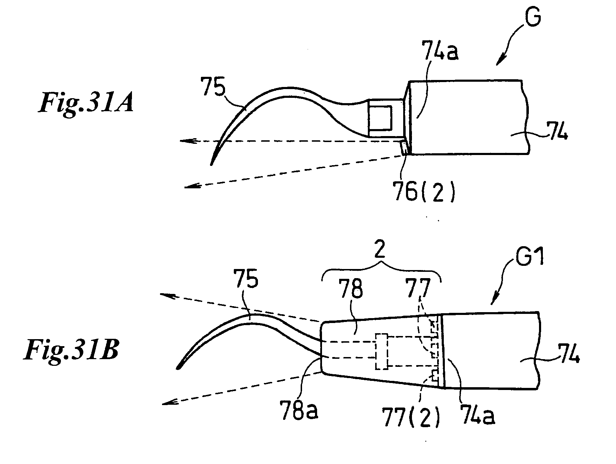

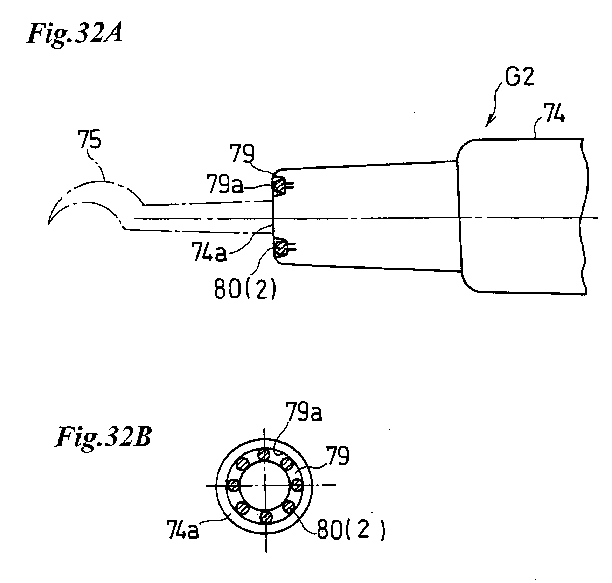

FIGS. 31A, 31B, 32A, and 32B show examples of medical treatment appliances as dental scaler handpieces. A scaler handpiece G of FIG. 31A has a scaler chip 75 detachably fitted to a head part 74a of a handpiece main body 74. To the fitting base of this scaler chip 75, the same bare-chip type LED 76 as described above having the exciting light emitting bare chip (exciting light emission section) and the white light emitting bare chip (illumination light emission section) is detachably fitted, thereby constructing the irradiation means 2. The field of irradiation from this irradiation means 2 is directed, as shown by arrows in the figures, to the tip side of the scaler chip 75.

The exciting light emitting bare chip described above is an emitter having a wavelength characteristic that fluorescence is generated when it irradiates the plaque or the tartar (lesion). The operator, before or during plaque or tartar removing operation, causes the exciting light emitting bare chip and the white light emitting bare chip to simultaneously emit lights and irradiates these lights to a subject portion to be treated. If there is the plaque or the tartar, fluorescence specific thereto is radiated and the plaque or the tartar is clearly visually recognized, and also the periphery of the plaque or the tartar (lesion) is illuminated through the irradiation of the illumination light and the relative position or degree of the plaque or the tartar in an outline image is adequately grasped, thus permitting the plaque or the tartar removing operation by the scaler chip 75 to be performed with favorable accuracy. The scaler chip 75 may be of the type which is vibrated by a vibration source, such as ultrasonic waves, built in the handpiece main body 74.

FIG. 31B shows, as an modified example of the example described above, a scaler handpiece G1 having, at the head part 74a of the handpiece main body 74, a plurality of LEDs as an emission section 77 so fitted as to surround the fitting base of the scaler chip 75. The emission section 77 includes at least the exciting light emitting LED (exciting light emission section) and the white LED for illumination light emission (illumination light emission section), which are capable of simultaneous emission. The numbers of exciting light emitting LEDs and the white LEDs for illumination light emission are set so that the ratio therebetween is substantially 6 to 2. Further, by adding the infrared light LED or the ultraviolet LED, through the light source selection switch, irradiation of only an illumination light may also be performed, and these LEDs may be selectively caused to emit light during scaling.

A light guide adapter 78 for collectively covering the emission section 77 composed of the plurality of LEDs described above is mounted at the head part 74a described above, and the irradiation means 2 is configured so that the light is irradiated, as shown by arrows of dashed lines, from an exit section 78a of this light guide adapter 78. The light guide adapter 78 is formed of a translucent molded body of heat resistant synthetic resin or a translucent optical fiber bundle, which permits protection of the emission section 77 and also permits performing sterilization treatment in an autoclave with the light guide adapter 78 left mounted.

The dental scaler handpiece G2 shown in FIG. 32 has the irradiation means 2 constructed with an emission section 80 composed of a large number of LEDs arrayed in a circumference groove 79 formed at the head part 74a of the handpiece main body 74 concentrically with the scaler chip 75. The emission section 80 includes at least the exciting light emitting LED (exciting light emission section) and the white LED for illumination light emission (illumination light emission section), which are capable of simultaneous emission. The numbers of the exciting light emitting LEDs and the white LEDs for illumination light emission are set so that the ratio therebetween is substantially 6 to 2. Further, by adding the infrared light LED or the ultraviolet LED, through a select switch, these LEDs may be selectively caused to emit light. On the inner wall surface of the circumference grooves 79, a reflective material 79a is so formed as to be deposited thereon.

Also in these dental scaler handpieces G1 and G2, as described above, if the exciting light emitting LED and the white LED for illumination light emission are caused to simultaneously emit lights and these lights are irradiated simultaneously to the subject portion to be treated, fluorescence specific to the plaque or the tartar is irradiated therefrom and also the periphery of the plaque or the tartar (lesion) is also illuminated through the irradiation of the illumination light and the relative position or degree of the plaque or the tartar in an outline image is adequately recognized, thus permitting the plaque or tartar removing operation by the scaler chip 75 to be performed with favorable accuracy.

### EIGHTEENTH EMBODIMENT

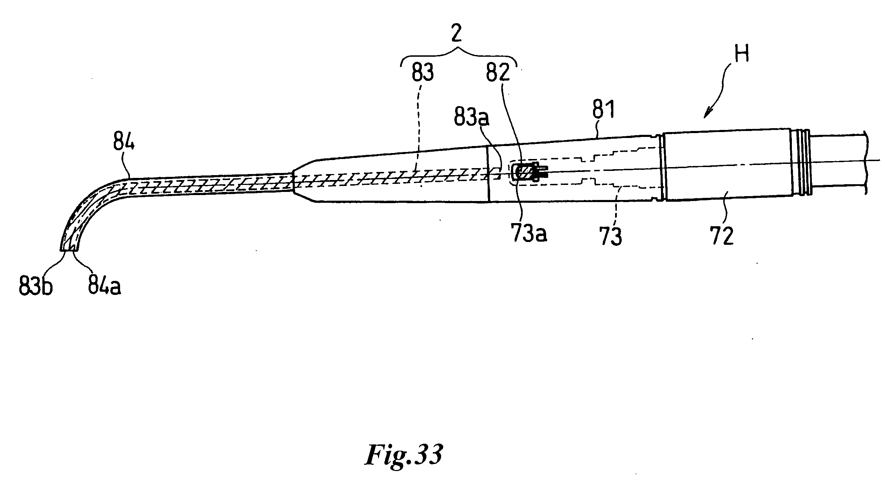

FIG. 33 shows an example of a medical treatment appliance as a dental light probe of a handpiece type. The dental light probe H in the figure has the irradiation means 2 composed of: the same bare-chip type LED as described above, as an emission section 82, disposed in the trunk of a handpiece main body 81; and a light guide 83 formed of a glass fiber or the like. The light guide 83 has an entrance surface 83a facing the light exiting section of the emission section 82 and an exit surface 83b extends to a tip end 84a of a probe 84, so that light guided through the light guide 83 is irradiated from this exit surface 83b toward a subject portion to be irradiated. The bare-chip type LED constituting the emission section 82 is provided with at least the exciting light emitting bare chip (exciting light emission section) and the white light emitting bare chip (illumination light emission section), which are capable of simultaneous emission.

The probe 84, if formed of a flexible tube or the like, appropriately bends together with the light guide 83 inserted therein, which is convenient for directing the irradiation light even to a narrow portion in the oral cavity. Moreover, the illustrated example shows that the base of the handpiece main body 81 is joined with the same hose side connector section 72 as shown in FIG. 30, and the emission section 82 is detachably stored and held in the depressed portion 73a formed at the tip end of the joint section 73. Therefore, if the joint section 73 including this emission section 82 is adapted to be equivalent to that shown in FIG. 30 and if the handpiece main body 62 of FIG. 30 and this handpiece main body 81 are adapted to be detachable by means of the joint section 73 from the connector section 72 for replacement, the tooth cutting handpiece and the light probe can be shared, which is extremely convenient. With this light probe, through operation of the light source selection switch, not shown, selection can be made from among: the exciting light, the illumination light, and a combination of the exciting light and the illumination light, in accordance with a purpose. Moreover, the tip end of the light probe is thin, thus permitting irradiation to be performed from behind a tooth to thereby achieve treatment through light transmission.

### NINETEENTH EMBODIMENT

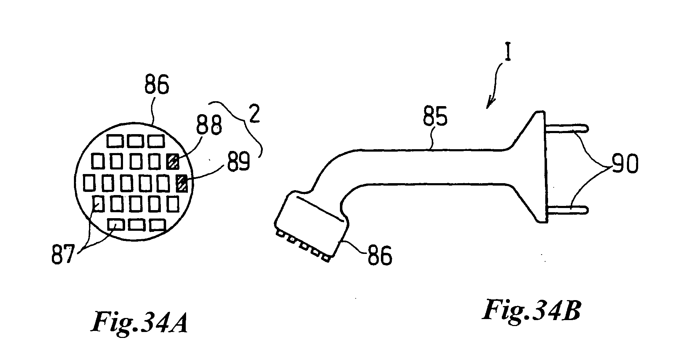

FIGS. 34A and 34B show examples of a dental photo-polymerizer as the medical treatment appliance. The dental photo-polymerizer I in the figure is composed of a polymerizer main body 85 and a polymerizer head 86. Mounted on the front surface of the polymerizer head 86 are a plurality of blue light LEDs 87, an exciting light emitting LED (exciting light emission section) 88, and an white light LED 89 (illumination light emission section) for illumination which are suitable for polymerization of photo-polymerization resin. This exciting light emitting LED 88 and the white light LED 89 for illumination constitute irradiation means 2. At the base end part of a polymerizer main body 85, a male terminal 90 for feeding power to these LEDs is led out, which is connected, upon photo-polymerization operation, to a socket cable (not shown) for photo-polymerization.

To perform photo-polymerization operation by using such a dental photo-polymerizer I, before the operation, the exciting light emitting LED 88 and the white light LED 89 for illumination are caused to simultaneously emit lights to simultaneously irradiate these exciting light and illumination light to the subject portion to be operated. If there is lesion in the subject portion to be operated, the fluorescence specific thereto is radiated by the exciting light, and also the periphery thereof is illuminated by the illumination light, and a fluorescence image of the lesion in an outline image can be clearly visually recognized. Therefore, after cutting and removing treatment or the like is performed on the lesion, photo-polymerizable resin is supplemented thereto and the blue light LED 87 for polymerization described above is caused to emit light to irradiate the photo-polymerization resin with this light, thereby achieving its polymerization. The blue light LED 87 for polymerization can be also used as the illumination light emission section, and their emission control is performed by the select switch, not shown.

### TWENTIETH EMBODIMENT

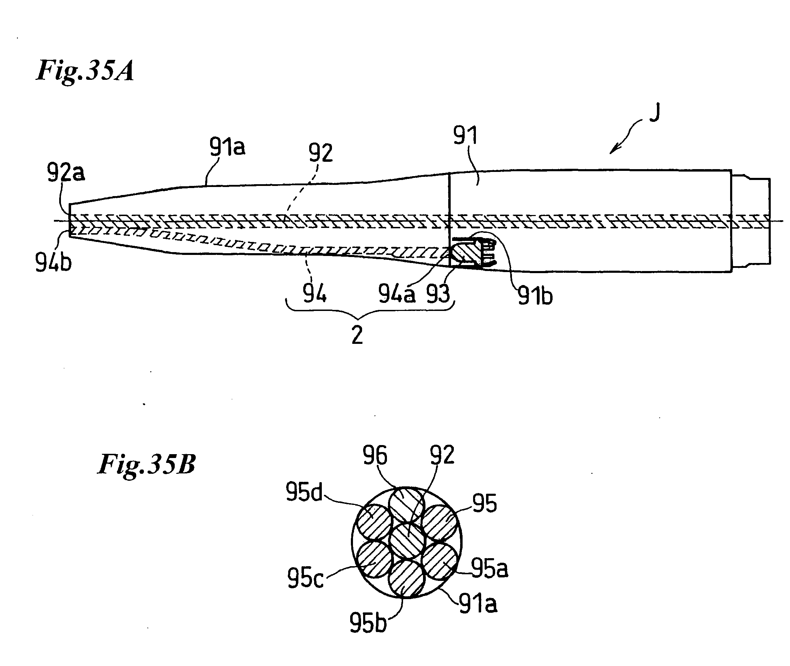

FIGS. 35A and 35B show examples of a medical treatment appliance as a dental laser treatment device. A dental laser treatment device J in the figure is composed of: a handpiece main body 91 connected to a light guide hose (not shown) having a laser light guide body fitted therein drawn from a laser oscillator (not shown) which is separately installed, and a laser light guide 92 which is fitted in the handpiece main body 91, which is joined with the laser light guide body in the light guide hose, and which extends to the tip end. The dental laser treatment device J is used for transpiration, incision, coagulation, haemostasis, warming, and pain relief of living tissue, cutting of a tooth, and the like in the oral cavity in a manner such that a laser light is caused to exit from a tip exit end 92a of the laser light guide 92 to be irradiated to the lesion.

In FIG. 35A, the irradiation means 2 is composed of the same bare-chip type LED as described above, as an emission section 93, disposed in the trunk of the handpiece main body 91; and a light guide body 94 which is formed of a glass fiber or the like fitted in a head part 91a detachably connected to the handpiece main body 91. The light guide body 94, when the head part 91a is connected to the handpiece main body 91, has an entrance surface 94a thereof facing the light exiting section of the emission section 93 and has an exit surface 94b thereof extending to the head of the head part 91a. An emitted light guided by the light guide body 94 is irradiated from this exit surface 94b toward the subject portion to be irradiated. The bare-chip type LED constituting the emission section 93 is detachably stored and held in a depressed portion 91b formed at the head part of the handpiece main body 91, and provided with at least the exciting light emitting bare chip (exciting light emission section) and the white light emitting bare chip (illumination light emission section) as described above, which are capable of simultaneous emission.

Upon laser treatment performed by using such a dental laser treatment device J, before the treatment operation or during the treatment operation, the exciting light emitting bare chip and the white light emitting bare chip are caused to simultaneously emit lights to simultaneously irradiate the exciting light and the illumination light to the subject portion to be treated. If there is lesion, fluorescence specific thereto is radiated and the lesion is clearly visually recognized, and also the periphery of the lesion is illuminated through the irradiation of the illumination light and thus the relative position or degree of the lesion in an image of healthy normal living tissue at the periphery of the lesion can be adequately grasped, thus permitting the laser treatment operation with favorable accuracy. A laser light is typically an invisible light, but the illumination light or the exciting light described above can also be used as a guide light for expressing a laser-irradiated portion. In the embodiment described above, the laser light guide body 92 and the light guide body 94 of the LED are separately provided, but the laser light guide body 92 can be adapted for use as a light guide for the LED.

FIG. 35B shows a modified example of described above, with the head part 91a of the handpiece main body 91 illustrated in horizontal cross section. Specifically, disposed in the handpiece main body 91 described above is at least irradiation means (not shown) composed of a plurality of emission sections including at least the exciting light emitting LED (exciting light emission section) and the white light LED for illumination (illumination light emission section), and a plurality of light guides 95, and 95a to 95d for irradiation, which are paired with these plurality of emission sections, are fitted in the head part 91a, each extending to the head part. In the handpiece main body 91, the imaging device, such as the CCD or the MOS (not shown), is built in, and in the head part 91a, a light guide body 96 for imaging is fitted which is paired with the light receiving section of this imaging device and which extends to the head thereof. Imaging means is composed of this light guide body 96 and the imaging device. The light guide bodies 95 and 95a to 95d for irradiation and the light guide body 96 for imaging described above are so bundled together as to surround the laser light guide body 92, and are fitted in the head part 91a.

Thus, from the exit end of the light guide bodies 95, and 95a to 95d for irradiation, as described above, an exciting light and an illumination light simultaneously exit, and are simultaneously irradiated to the subject portion to be treated. The same radiation light image described above from the subject portion to be treated based on this irradiation is guided by the light guide body 96 for imaging, and formed on the imaging device. In the imaging device, this radiation light image is converted into an electrical signal, and then transmitted to a personal computer, not shown, or the like, where it is appropriately displayed on the display, printed out, or stored as carte information. Therefore, this can be used as information for achieving communication with a patient, and is extremely practical as diagnostic information.

### TWENTY-FIRST EMBODIMENT

FIG. 36 shows an electrical circuit by which the exciting light and the white light are simultaneously irradiated and the light amount balance between the exciting light and the white light is adjusted by the variable resistors (light amount adjustment means) in the embodiment described above. Numeral 97 denotes a light amount adjusting variable resistor for the white light emitting LED (illumination light emission section) 99, and numeral 98 denotes a light amount adjusting variable resistor for an exciting light emitting LED (exciting light emission section) 100. By these variable resistors, the currents running through the respective LEDs are adjusted to thereby adjust the light amounts of the respective emission sections. The adjustment by each of the variable resistors described above permits switching the light amount balance between the illumination light and the exciting light while simultaneously irradiating the illumination light and the exciting light, thereby simultaneously observing the healthy normal tissue at the periphery of the lesion and the lesion with optimum light amount balance.

Here, by operating the variable resistors 97 and 98 for the respective LEDs 99 and 100 to adjust the light amounts of the respective LEDs until reaching zero, in addition to a mode for the simultaneous irradiation described above, a mode for only white light (illumination light) irradiation or a mode for only exciting light irradiation can be selected and thereby forming a selection means. Moreover, at shipment from a plant, each of the light amount adjusting variable resistors 97 and 98 for the respective LEDs can be operated so that they are provided and fixed with irradiation modes at the optimum setting described above before shipped. Desirably, the amount of the white light as the illumination light is set smaller than that of the exciting light, which permits avoiding fluorescence from being buried in the illumination light and also permits both the lesion and the healthy normal tissue at the periphery of the lesion to be simultaneously visually recognized. It is desirable that such light amount setting be initial setting at the shipment from the plant.

Moreover, in the embodiments described above, the white light as the illumination light is used, but a reddish color light or a yellowish color light may be used when necessary. The adjustment of the variable resistors described above allows switching of the light amount balance between the illumination light and the exciting light while simultaneously irradiating the illumination light and the exciting light, thereby permitting both the healthy normal tissue at the periphery of the lesion and the lesion to be simultaneously observed with optimum light amount balance.

### TWENTY-SECOND EMBODIMENT

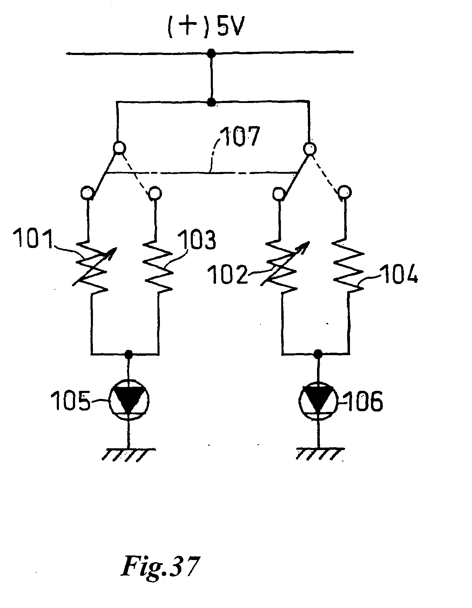

FIG. 37 is a circuit diagram designed so that, at shipment from a plant, optimum initial setting can be made, and light amount adjusting variable resistors can be arbitrarily operated by the user to thereby optionally adjust light amounts of the white light emitting LED (illumination light emission section) 105 and the exciting light emitting LED (exciting light emission section) 106. The select switch 107, as shown by solid lines and dashed lines in the figure, can be switched between an at-shipment initial setting side and a user's arbitrary adjustment side. When the select switch 107 is switched to the user's arbitrary adjustment side as shown by the solid lines, the variable resistor 101 for the white LED adjustment and the variable resistor 102 for the exciting light adjustment can be individually and arbitrarily adjusted. For optimum initial setting at shipment from the plant, adjustment to optimum balance between the exciting light and the white light can be achieved through a fixed resistor 103 for the white LED adjustment and a fixed resistor 104 for exciting light adjustment by switching the select switch 107. The user can switch between the at-shipment initial setting and the user' arbitrary adjustment through operation of the select switch 107. These light amount adjustment means are applicable to any one of the embodiments described above. The adjustment of each of the variable resistors 101 and 102 permits switching the light amount balance between an illumination light and an exciting light while simultaneously irradiating the illumination light and the exciting light. This enables the healthy normal tissue at the periphery of the lesion and the lesion to be simultaneously observed with optimum light amount balance. The select switch 107 may be provided in the handpiece main body casing 1 or in the control box H.

### TWENTY-THIRD EMBODIMENT

FIG. 38 shows one example of irradiation light selection means (corresponding to the light source selection switch 7 of FIG. 1). Specifically, the irradiation light selection means 7 is irradiation driving means which constitutes the irradiation means 2 composed of four types of emission sections (a plurality of emission sections emitting lights of mutually different wavelengths) 2A to 2D comprised of LEDs for the infrared light (first light source), the white light (illumination light emission section, second light source), the ultraviolet light part 1 (exciting light emission section, third light source), and the ultraviolet light part 2(exciting light emission section, fourth light source), for selectively driving any one (or more) of these plurality of emission sections 2A to 2D. The irradiation light selection means 7 is provided with: four analog switches sw1 to sw4 connected between a power source 108 and the emission sections 2A to 2D; four light source selection switches hs1 to hs4; and a switch control section 109. Moreover, the variable resistors for current adjustment described in FIGs. 36 and 37 can be inserted in the circuit to thereby adjust the light amount of the light source.

ON operation of the first light source selection switch h1 permits the first analog switch sw1 to activate the infrared light LED 2B, and in the same manner, ON operation of the second light source selection switch hs2 activates the white light LED 2A, ON operation of the third light source selection switch hs3 activates the ultraviolet light part 1 LED 2C, and ON operation of the fourth light source selection switch hs4 activates the ultraviolet light part 2 LED 2D. This method permits selectively irradiating irradiation lights of arbitrary types individually or in an overlapping manner.

Moreover, simultaneous ON operation of the second light source selection switch hs2 for the white light LED 2A and the third light source selection switch hs3 or the fourth light source selection switch hs4 for the ultraviolet light part 1 LED 2C or the ultraviolet light part 2 LED 2D permits simultaneous irradiation of the illumination light and the exciting light. Through such simultaneous irradiation, as described above, a fluorescence image of the lesion obtained through the irradiation of the exciting light and a reflection light image of the healthy normal tissue at the periphery of the lesion obtained through the irradiation of the illumination light are clearly visually recognized, thus permitting the position and degree of the lesion to be adequately comprehended. Thus, not only irradiation of the illumination light only, irradiation of the exciting light only, and simultaneous irradiation of the illumination light and the exciting light can be selectively performed, but also the light amounts of the exciting light and the illumination light can be adjusted at shipment as the initial setting selection and also adjusted individually by each user.

If sequence such that the illumination light and the exciting light are irradiated in a time-shared manner at a short interval is previously defined and an exclusive switch therefor is provided so that the irradiation in the time-shared manner is performed through operation of this switch; when the subject portion to be irradiated is visually recognized directly, due to a residual image phenomenon of the retina and also through image processing via imaging means, the fluorescence image and reflection light image described above are superposed one on another, thus providing the same effect as is provided by simultaneous irradiation. Moreover, in combination with the reflection light image obtained through irradiation of the infrared light, the diagnostic image information can be provided.

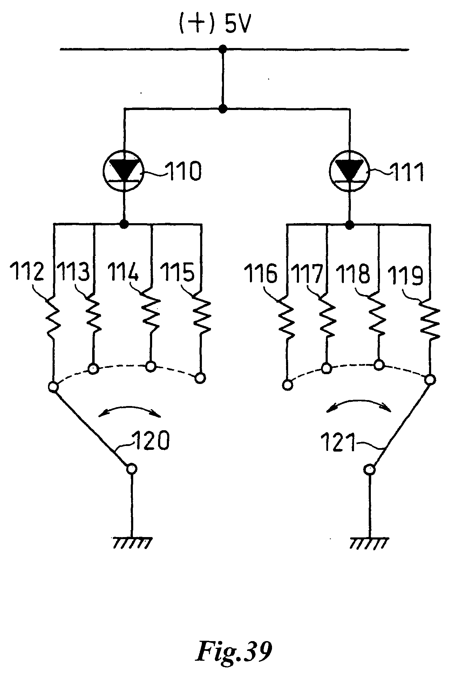

FIG. 39 shows another example of light amount adjustment means. In this example, the light amount adjustment means for the irradiation light is constructed with an illumination light irradiation LED 110, such as the white LED, connected to a rotary switch 120 which is selectively connected to resistors 112, 113, 114, and 115 of different resistance values for the purpose of adjusting the light amount and also with an exciting light irradiation LED connected to a rotary switch 121 which is selectively connected to resistors 116, 117, 118, and 119 of different resistance values for the purpose of adjusting the amount of the exciting light. For example, the resistance values of the resistors 112, 113, 114, and 115 for illumination light amount adjustment can be appropriately set to provide light amounts of 2%, 35%, 75%, and 100%, respectively. The resistance values of the resistors 116, 117, 118, and 119 for exciting light amount adjustment can also be appropriately set to provide light amounts of 2%, 35%, 75%, and 100%, respectively.

Through such configuration, the rotary switches 120 and 121can be appropriately selected in accordance with a purpose to thereby adjust the light amount balance between the illumination light and the exciting light while simultaneously irradiating the illumination light and the exciting light. To visually recognize condition of the lesion, such as caries, the resistor 119 for light amount of 100% is selected for the exciting light, the exciting light is irradiated a little strongly, and the resistor 112 for the light amount of 2% is selected for the illumination light. As a result, the healthy normal tissue at the periphery of the lesion is dark, but distribution of the lesion generated by fluorescence can be visually recognized clearly. To focus on the healthy normal tissue at the periphery of the lesion, the resistor 119 for the light amount of 100% is selected by the rotary switch 121 for the exciting light, and the resistor 115 for the light amount of 2% is selected by the rotary switch 120 for the illumination light.

The light amount adjustment means for switching the light amount balance between the illumination light and the exciting light of irradiation means is configured by using the rotary switches and the resistors in the description above, although not limited thereto, and thus various well-known circuits can be used. Moreover, under the condition that combinations of light amounts of the illumination light source and the exciting light source are previously set in accordance with individual cases or application purposes, the combination may be switched by selecting the selection switch in accordance with the individual case or the application purpose so as to provide arbitrarily set light amounts for the illumination light source and the exciting light source.

In the embodiment described above, the irradiation means may be turned on and off by a foot pedal switch. Moreover, the living body observing apparatus, the intraoral imaging apparatus, or the medical treatment appliance of the present invention are very effective for dental use in particular, although not limited thereto, and thus can be widely adopted for medical use in otology, internal medicine, dermatology and the like. Furthermore, these apparatuses can be widely used at general households, and thus can serve as reference for caries prevention and skin care.

## Claims

1. A living body observing apparatus comprising irradiation means including an exciting light emission section emitting an exciting light for causing lesion to radiate fluorescence and an illumination light emission section emitting an illumination light for illuminating the periphery of said lesion, wherein said irradiation means simultaneously irradiates the illumination light and the exciting light.

2. The living body observing apparatus according to claim 1,
wherein said irradiation means comprises light amount adjustment means for adjusting an irradiation amount of at least one of the illumination light and the exciting light.

3. The living body observing apparatus according to claim 1 or 2, comprising means for selecting any one of a mode in which the exciting light emission section and the illumination light emission section are simultaneously caused to emit the light for the irradiation; a mode in which only the illumination light emission section is caused to emit the light for the irradiation; and a mode in which only the exciting light emission section is caused to emit the light for the irradiation.

4. The living body observing apparatus according to any one of claims 1 to 3,
wherein the illumination light emission section of the irradiation means emits one or more of white light, monochromatic light, infrared light, and ultraviolet light.

5. The living body observing apparatus according to any one of claims 1 to 4,
wherein said exciting light emission section of the irradiation means is comprised of any one of an LED, a laser diode, a semiconductor laser, a solid state laser oscillator, and a laser oscillator.

6. The living body observing apparatus according to any one of claims 1 to 4,
wherein said exciting light emission section of said irradiation means is comprised of combination of any one of a halogen lamp, a metal halide lamp, a xenon lamp, a krypton lamp, a mercury lamp, and a sodium lamp with an irradiation filter.

7. The living body observing apparatus according to any one of claims 1 to 6,
wherein a wavelength of the exciting light irradiated from the irradiation means is any one of 365 ± 30 nm, 400 ± 30 nm, and 470 ± 30 nm.

8. The living body observing apparatus according to any one of claims 1 to 7,
wherein an amount of light emitted from the exciting light emission section is larger than that of light emitted from the illumination light emission section.

9. The living body observing apparatus according to any one of claims 1 to 9,
wherein the irradiation means is detachably fitted to said body.

10. The living body observing apparatus according to any one of claims 1 to 9,
wherein the exciting light emission section and/or the illumination light emission section of the irradiation means emits a plurality of types of lights of mutually different wavelengths, and further comprises selection means for selecting one or more of the plurality of types of lights.

11. The living body observing apparatus according to claim 10, further comprising irradiation driving means for causing the selected light to be emitted and irradiated.

12. The living body observing apparatus according to any one of claims 1 to 11, further comprising imaging means for imaging a radiation light image formed of a fluorescence image from the lesion and/or an illumination light image from the periphery of the lesion.

13. The living body observing apparatus according to claim 12,
wherein said imaging means comprises a light receiving filter for cutting off the exciting light.

14. An intraoral imaging apparatus comprising a main body including an imaging means and the irradiation means according to any one of claims 1 to 11 which is provided in the main body.

15. An intraoral imaging apparatus comprising: a main body including imaging means; and irradiation means comprising an exciting light emission section emitting an exciting light for causing lesion to radiate fluorescence and an illumination light emission section emitting illumination light for illuminating the periphery of the lesion, wherein:
said irradiation means and said imaging means are provided in the main body;
said irradiation means simultaneously irradiates the illumination light and the exciting light; and wherein
an amount of the light emitted from said exciting light emission section is larger than an amount of the light emitted from said illumination light emission section.

16. The intraoral imaging apparatus according to claim 14 or 15,
wherein said imaging means comprises a light receiving filter for cutting off the exciting light.

17. The intraoral imaging apparatus according to claim 16,
wherein the light receiving filter is detachably fitted to the main body of said apparatus.

18. The intraoral imaging apparatus according to any one of claims 15 to 17,
wherein said irradiation means comprises light amount adjustment means for adjusting an irradiation amount of at least one of the illumination light and the exciting light.

19. An intraoral imaging apparatus comprising a main body comprising imaging means, and irradiation means comprising an exciting light emission section emitting an exciting light for causing lesion to radiate fluorescence and an illumination light emission section emitting a illumination light for illuminating the periphery of lesion, wherein:
the irradiation means and the imaging means are provided in the main body of said apparatus; and
said irradiation means comprises light amount adjustment means for simultaneously irradiating the illumination light and the exciting light and further switches irradiation amount balance between the illumination light and the exciting light.

20. A medical treatment appliance into which the living body observing apparatus or the intraoral imaging apparatus according to any one of claims 1 to 19 is integrally incorporated.
